# EUROPEAN PATENT SPECIFICATION

(11) **EP 1 109 932 B1**
(45) Date of publication and mention of the grant of the patent: **16.06.2004**
(21) Application number: 99935912.8
(22) Date of filing: 23.07.1999
(51) Int. Cl.: C12Q 1/68

(54) **METHODS FOR DETECTING AND MEASURING SPLICED NUCLEIC ACIDS**
VERFAHREN ZUR ERKENNUNG UND MESSUNG VON GESPLEISTEN NUKLEINSÄUREN
PROCEDES POUR DETECTER ET MESURER DES ACIDES NUCLEIQUES EPISSES

(30) Priority: 23.07.1998 US 121239
(43) Date of publication of application: 27.06.2001
(73) Proprietor: Gen-Probe Incorporated, San Diego, CA 92121-4362 (US)
(72) Inventor: HARVEY, Richard, C., Thousand Oaks - California 91360 (US); EASTMAN, Paul, S., Moraga, CA 94556 (US)
(74) Representative: Viering, Hans-Martin, Dipl.-Ing.
(86) International application number: PCT/US1999/016832
(87) International publication number: WO 2000/005418

(56) References cited:
- WO-A-97/08339
- DE-A- 4 447 015
- US-A- 5 057 410
- US-A- 5 480 784
- US-A- 5 614 391
- SOOKNANAN R ET AL: "DETECTION AND DIRECT SEQUENCE IDENTIFICATION OF BCR-ABL MRNA IN PH+CHRONIC MYELOID LEUKEMIA" EXPERIMENTAL HEMATOLOGY,US,NEW YORK, NY, vol. 21, no. 13, December 1993 (1993-12), page 1719-1724 XP000600204 ISSN: 0301-472X cited in the application

## Description

### Field of the Invention

This invention relates to detection and measurement of nucleic acids derived from biological sources, and specifically relates to methods for specifically detecting spliced mRNA, including normally spliced mRNA resulting from intracellular processing and atypically spliced mRNA resulting from chromosomal translocation events such as occur in some cancerous cells.

### Background of the Invention

In eukaryotes, the genetic information in DNA is present in chromosomes contained within a nucleus. Some viruses contain chromosome-like genetic structures. The genetic information contained within one strand of DNA depends on the sequence of bases (i.e., "base sequence" or "nucleotide sequence") where the bases are adenine (A), guanine (G), cytosine (C) and thymine (T). DNA encodes complementary messenger ribonucleic acid (mRNA) sequences, in which T is replaced with uracil (U) and the 5' to 3' nucleotide sequence specifies the amino acid sequence of the encoded protein. Eukaryotic genes generally include noncontiguous coding regions ("exons") separated by intervening non-coding regions ("introns"). Nuclear transcription of a gene synthesizes a precursor mRNA (pre-mRNA, including introns and exons) complementary to the coding DNA strand. Normal processing of pre-mRNA eliminates introns by cleaving and splicing the RNA to covalently link the exons and concurrently excise the introns. The resulting mature nuclear mRNA exits into the cytoplasm where translation into protein occurs.

Nucleic acid splicing also occurs in the life cycle of many viruses. Some viruses integrate into a host cell's DNA as a provirus using a splicing mechanism in which the host DNA is cleaved and the viral nucleic acid is inserted and ligated to the host DNA. Viral insertion often occurs at a specific locus or related loci, characteristic of the virus or viral family, in the host chromosome. Pathogenic proviruses present in a target cell population are often associated with a specific condition or disease. Insertion of a provirus within a chromosomal exon or near an intron-exon border may lead to the production of an abnormal version of a normally transcribed mRNA and/or translated protein, with subsequent deleterious effects on the cell.

Molecular biology methods have been used to investigate the genetic basis of disease and identify molecular events, e.g., aberrant genetic splicing, deletions, insertions, substitutions and amplifications, that are responsible for some diseases, such as cancer. Some aberrant genetic splicing events are nonrandom and characteristic of particular diseases.

Chromosomal translocations are genetic recombination events associated with certain cancers, such as some leukemias and lymphomas. In some translocations, two different chromosomes reassemble by reciprocal genetic exchange between the chromosomes forming two hybrid chromosomes, each including portions of two normal chromosomes. Other translocations are intrachromosomal recombinants, in which normally noncontiguous regions of the same chromosome are joined. A "breakpoint junction" of a translocation refers to a joining point of sequences derived from normally separated chromosomal locations. Breakpoint junctions can be clustered in conserved locations within one or both of two chromosomes. Translocations occurring within a genetic coding region may result in abnormal mRNA having discrete regions, e.g., a 5' portion derived from one chromosomal location and a 3' portion derived from another chromosomal location. Such abnormal transcripts are known as "fusion transcripts" or "fusion mRNA."

A family of translocations are characteristic of patients having chronic myelogenous leukemia ("CML"). CML-associated translocations occur between human chromosomes 9 and 22 (referred to as "t(9:22)"), and the resulting shortened chromosome 22 is known as the Philadelphia chromosome (or Ph¹). The t(9;22) events link portions of the *abl* gene of chromosome 9, and the "breakpoint cluster region" or *bcr* gene of chromosome 22. A cDNA prepared from a fusion mRNA (of about 8 kb) isolated from a Ph¹-positive cell line has been sequenced, revealing a translocation between the *abl* exon 2 and the *bcr* b3 region that encodes a fusion protein having a tyrosine kinase activity (Shtivelman *et al., Nature* 315:550-554, 1985). Antibody-detection of an altered Abl protein having a molecular weight higher than that of normal Abl protein has been used to detect Ph¹-positive cells, diagnostic of CML (see U.S. Pat. No. 4,599,305 to Witte *et al.).*

Another translocation between chromosomes 22 and 9, within a *bcr* region about 50 kb 5' to the CML-associated *bcr* region, is characteristic of acute lymphoblastic leukemia (ALL). ALL-associated translocations occur within the putative first intron of the *bcr* region, producing a chimeric mRNA that splices the *bcr* b1 of chromosome 22 and the *abl* exon 2 of chromosome 9, which in turn produces a fusion protein (Hermans *et al., Cell*51:33-40, 1987). ALL-associated translocations on chromosome 22 have been detected with probes specific for a portion of the *bcr* gene (see European Pat. Pub. No. EP 0 364 953 by Nakamura *et al.).*

Chromosomal translocations involving human chromosomes 8 and 21 have been associated with about 40% of pediatric acute myelogenous leukemia ("AML") having the FAB-M2 morphology. The breakpoints on both chromosomes are variable, but generally result in a common fusion transcript containing 5' portions of the AML1 gene of chromosome 21 and 3' portions, of the ETO gene of chromosome 8, which produce a fusion protein (see U.S. Pat. No. 5,580,727 to Ohki et *al*.).

Other chromosomal translocations associated with diseases such as lymphomas and leukemias are the ALL-associated t(15;17) translocation, and the AML-associated t(12;21), t(4;11) and the t(1;19) translocations.

Detection of chimeric DNA and/or RNA and/or fusion proteins associated with abnormal conditions or diseases such as those exemplified above is useful for confirming an initial diagnosis, for monitoring a patient's response to treatment, and for providing early warning of recurrence of the disease after remission. The ability to detect chimeric nucleic acids or proteins has been limited by the extremely small numbers of cells present at different stages of a disease, e.g., following remission or in a non-acute phase. A patient's prognosis for conditions associated with chromosomal translocations, including recurrence of the condition, is usually more favorable with early diagnosis. Generally, immunological techniques are not sensitive enough to detect very small amounts of analyte in a sample.

Methods for the diagnosis of conditions associated with chromosomal translocations, such as CML and ALL have been reported. U.S. Pat. No. 4,681,840 to Stephenson *et al*. and PCT Pub. No. WO 85/03297 disclose DNA and hybridization methods to detect CML-associated Ph¹ abnormalities using a chromosomal DNA-derived probe complementary to the *bcr* region. CML-associated t(9:22) translocations that join *bcr* b2 or b3 with *abl* exon 2 can be detected by amplifying the fusion mRNA and then hybridizing synthetic oligonucleotide probes specific for these spliced sequences to the amplified nucleic acid (see U.S. Pat. No. 4,874,853 to Rossi *et al.* and European Pat. Pub. No. 0338713). Methods of detecting unique aberrant gene transcripts by hybridizing the RNA with one or more synthetic DNA oligonucleotides complementary to sequences encoded by the Ph' chromosome have been described in U.S. Pat. No. 4,999,290 to Lee. In these methods, an RNA-DNA heteroduplex is formed that is resistant to enzymatic degradation, followed by polymerase chain reaction (PCR) amplification and DNA detection to indicate the presence of aberrant gene transcripts . The complementary DNA oligonucleotides include *bcr* b2 and/or b3 and *abl* sequences. DNA sequences and hybridization methods for detecting and identifying chromosomal aberrations in tumor DNA containing the ALL-1 breakpoint region of human chromosome 11 (e.g., t(9;11) translocations) are known (see U.S. Pat. Nos. 5, 567,586 and 5,633,136 to Croce *et al.)*

Methods that use junction probes to detect translocations can detect only DNA or mRNA resulting from specific fusion events because each probe is directed to a specific breakpoint junction. For translocations, such as CML-associated events, that may use any one of many breakpoints to occur within a small *bcr* region, fusion mRNA detection would require use of many different breakpoint junction probes. Also, point mutations that may limit probe hybridization are sometimes located within a few bases of a splice junction. Moreover, chromosomal rearrangements such as deletions or insertions within the bcrgene may produce less-common CML-associated abnormal transcripts that would not be detected by using a breakpoint junction probe.

U.S. Pat. No. 5,487,970 to Rowley *et al.* discloses methods for detecting chromosome 11 (11q22) translocations, using a breakpoint junction probe, or employing fluorescent *in situ* hybridization (FISH). In the FISH method, two fluorescently-labeled probes directed to 11q22 chromosomal regions flanking a commonly-found breakpoint are hybridized to chromosomes *in situ* which are then are obsenred using fluorescence microscopy. Cells in which the label is present only on chromosome 11 are classified as normal, whereas cells in which the label appears on different chromosomes are identified as possessing a translocation. This method requires screening a relatively large cell population unless the translocation is present in many cells in the sample. The probes include restriction endonuclease fragments that can hybridize to chromosomal translocations involving the chromosome 11 ALL-1 gene (using FISH or Southern blotting). Other FISH methods that detect CML-associated translocations use probes derived from species-specific DNA regions between repeat segments ("inter-Alu" sequences) as described in U.S. Pat. No. 5,538,869 to Siciliano *et al.*

The aforementioned methods rely on direct detection of the chimeric RNA or DNA by nucleic add hybridization without signal or target amplification. Therefore, these detection methods require a relatively large number of target cells bearing the chromosomal rearrangement in the sample, e.g., tissue, blood, or other body fluid. Large numbers of abnormal cells, however, generally are not present in a sample when the disease appears to be in remission or is in a chronic non-acute state. Thus, when a sufficient number of cells bearing genetic abnormalities are present to permit direct detection, a patient's treatment options may be severely limited.

Other methods of nucleic acid detection use amplification to obtain multiple copies of a target nucleic acid (either or both of the complementary strands) or a reporter molecule to increase detection sensitivity. A variety of nucleic amplification methods are well known (e.g., see Persing, D.H., ''In Vitro Nucleic Acid Amplification Techniques" in *Diagnostic Medical Microbiology: Principles and Applications* 51 (Persing *et al.,* Ed., 1993)), as described briefly below. Eskola et al. (Clinical Biochemistry, Vol. 27, No. 5, pp. 373-379, 1994) discloses a method of detecting translocations between the human *abl* gene of chromosome 9 and the bcr gene of chromosome 22 by using a reverse transcriptase polymerase chain reaction (RT-PCR) to amplify part of the translocation sequence, followed by the detection of the amplified nucleic acid using a method that requires at least two probes for detection. One of said two probes is biotinylated and binds 5' of the translocation splice junction and the second probe is Eu-labelled and binds 3' of a splice junction. The biotinylated probe is used to bind the hybrid complexes to a streptavidin-coated well and the Eu-labelled probe is used to detect the complexes by detecting fluorescence in the washed wells.

WO 97/08339 discloses methods of detecting *bcr-abl* translocations associated with CML by amplifying RNA sequences by using amplification primers that hybridise to positions flanking the splice junction and a transcription associated method (e.g., 3SR or NASBA), and then detecting the reaction product, if any, after capture onto a solid phase such as a micro-well by means of capture agents. The detection step uses a capture agent and a detector agent to form a target/capture agent/detector agent complex bound to a well and unbound material is washed away. In some embodiments of WO 97/08339, the capture agent and detector agent binding steps may be sequential, in which the reaction product is coupled to the solid phase, washed, and then the detector agent is bound. In another sequential method disclosed in WO 97/08339, the amplified sample is bound to the detector agent and the sample/detector agent complex is then bound to the micro-well coated with capture agent. Another embodiment of WO 97/08339 uses simultaneous binding, in which the amplified sample is simultaneously mixed with the capture agent and the detector agent and the target/capture agent/detector agent complex is then applied to a micro-well.

DE-A-4447015 describes a procedure suitable for isolating and purifying nucleic acids from small amounts of highly contaminated materials comprising: (a) lysing the material with a buffer containing a chaotropic salt with a high ionic strength; (b) incubating the lysate with a finely divided, non-porous, non-structured, homogeneous silica carrier having a particle size of 7-40 nm and a surface area of 50-300 m<2>/g; (c) separating the carrier-bound nucleic acid from the lysate; (d) washing the carrier with a buffer, and (e) eluting the nucleic acid with a low-salinity buffer.

Polymerase chain reaction (PCR) permits detection of small amounts of DNA present in a sample by amplifying the DNA target using two or more primers and a repeated cydes of thermal denaturation, primer annealing and DNA synthesis (U.S. Pat. No. 4,683,195 to Mullis *et al.; PCR Protocols: A Guide to Methods and Applications,* 1990, Innes, M.A. *et al.,* Eds. (Academic Press, Inc., San Diego, CA)). Generally, a first primer hybridizes to a specific region of the target nucleic acid, and a second primer hybridizes to the opposite strand of the target DNA 5' to the binding site of the first primer. Then in a series of synthesis steps, polymerase produces primer extension products that exponentially amplify the region between the primers.

The ligase chain reaction (LCR) uses two complementary sets of short DNA oligonucleotides that hybridize to adjacent regions of a target nucleic acid and the oligonucleotides are then covalently linked by using a DNA ligase (see European Pat. No. 0320308). By employing repeated cycles of thermal denaturation, hybridization and ligation, an accumulated double-stranded ligated oligonucleotide product can be detected to indicate the presence of the target nucleic acid in a sample.

Strand displacement amplification (Walker *et al.,* 1992, *Proc. Natl. Acad. Sci. USA* 89:392-396), employs oligonucleotide primers that contain restriction endonuclease cleavage sites and hybridize to opposite strands of a target nucleic acid duplex on either side of the sequence to be amplified. DNA polymerase-mediated primer extension using three deoxynucleoside triphosphates (dNTP) and a single dNTP[α]S produces a duplex hemiphosphorothioated primer extension product, which is nicked rather than cut by restriction endonuclease. Then, the 3' end of the nick is extended by a DNA polymerase that simultaneously displaces the non-hemiphosphorothioated strand. Each displaced strand of one sense can serve as a template for the binding of oligonucleotide primers of the opposite sense, resulting in the geometric accumulation of double stranded nucleic acids.

Another nucleic acid amplification method employs an RNA replicase (Qβ replicase) capable of amplifying the probe itself (see U.S. Pat. 5,112,734 to Kramer *et al.).*

Transcription based amplification systems employ an RNA polymerase to make RNA transcripts of a target region (see U.S. Pat. Nos. 5, 480,784 and 5,399,491 to Kacian *et al.).* One method, termed transcription-mediated amplification (TMA), uses a promoter primer that hybridizes to a target nucleic acid. In the presence of a reverse transcriptase, double-stranded DNA is formed, forming a double-stranded promoter. Then, an RNA polymerase produces RNA transcripts which become templates for further rounds of TMA in the presence of a second primer capable of hybridizing to the RNA transcripts. Unlike PCR and other methods that require heat denaturation, TMA is an isothermal amplification method that uses an RNAse H activity to digest the RNA strand of an RNA-DNA hybrid, thereby making the DNA strand available for hybridization with a primer or promoter primer. Generally, the RNAse H activity is supplied by a retroviral reverse transcriptase provided for amplification.

Chromosomal translocations and their transcription products have been detected using methods that include nucleic acid amplification. A PCR-based method to detect cells containing genomic DNA having t(14q32;18q21) chromosomal translocations associated with follicular lymphomas, and especially with minimal residual or relapse disease, by amplifying sequences surrounding a breakpoint cluster region, have been described in U.S. Pat. No. 5,024,934 to Lee. PCR primers and cDNA amplification methods for identifying t(9;11) translocations in a patient's tissue have been described in U.S. Pat. No. 5,633,135 to Croce *et al.* Methods for detecting carcinoma metastases (one in 10,000 to 100,000 cells) involving PCR amplification of target nucleic acids have been described by Green *et al.* in European Pat. Publication No. EP 520794.

Similarly, PCR amplification has been used to detect chimeric mRNA associated with ALL (U.S. Pat. No. 5,057,410 to Kawasaki *et al.)* and RNA transcripts originating from t(8;21) translocation in leukemic cells (U.S. Pat. No. 5,547,838 to Nisson *et al.).* U.S. Pat. No. 5,057,410 discloses PCR methods to detect chimeric mRNA containing exon-exon junctions. In this method, mRNA extracted from the target cell is reverse transcribed to produce cDNA that is amplified by PCR to make a double-stranded DNA amplification product. This amplification product is then denatured and one of the resulting strands is detected by hybridizing an oligonucleotide probe to the breakpoint junction. Individual probes hybridize to distinct *bcr-abl* junction species associated with CML and ALL.

Sooknanan *et al. (Experimental Hematol.* 21:1719-1724,1993) described a method for the detection of *bcr-abl* mRNA characteristic of CML by extracting RNA from FICOLL™-fractionated peripheral blood leukocytes and amplifying a target nucleic acid using an isothermal amplification procedure involving transcription. Serial reactions using four amplification primers (a primary pair in the first reaction and a nested pair in the second reaction) were essential for amplification and detection. The amplified nucleic acid produced by this method was of the same sense as the original mRNA and was detected using two different *bcr-abl* junction probes for detecting two splice junctions. Probes to the breakpoint junctions distinguished normal *bcr* and/or *abl* mRNA from chimeric *bcr-abl* mRNA in a patient sample.

PCR-based methods have been used for detecting other translocations associated with cancers or their transcription products. Probes and PCR primers for detecting t(2;13) translocations associated with alveolar rhabdomyosarcoma have been described in U.S. Pat. No. 5,650,278 to Barr *et al.* A nucleic acid sequence encoding a fusion protein (anaplastic lymphoma kinase or ALK) associated with t(2;5) lymphomas has been disclosed in U.S. Pat. No. 5,529,925 to Morris *et al*.

There remains a need in the art for a simple, sensitive, and rapid method for the detection of chimeric nucleic acids, particularly chimeric mRNA obtained from biological samples such as blood, marrow, plasma, biopsy tissue, sputum, urine, feces, semen or other body fluids. Preferably, such methods would require a minimum of technical expertise by laboratory personnel and a minimum of specialized laboratory equipment (*e.g*., centrifuges, thermocycling and electrophoresis equipment), and would use relatively low-cost reagents. Furthermore, there exists a need for an assay for detecting chimeric mRNA that would provide a result capable of interpretation with a minimum of qualification by, for example, reducing the possibilities of false positive and false negative results.

Also needed in the art is a simple and rapid method of preparing cytoplasmic nucleic acids, particularly mRNA, for use as a potential target in diagnostic nucleic acid hybridization assays or as a template for nucleic acid amplification. Such simplified preparative methods reduce the need for exhaustive extraction and purification procedures.

A crucial element of methods of detecting RNA (or amplification products made therefrom), particularly mRNA, is the manner of extracting RNA from the cells. Because of the ubiquitous presence of various RNases, extraction methods must preserve the small amounts of target RNA that may be present in a sample. Extraction methods that liberate all nucleic acids (including nuclear nucleic acids) from the target cell, produce samples that contain not only the target mRNA but also DNA encoding it which can produce false positive results in many nucleic acid-based assays.

Known extraction techniques generally use a chaotropic agent such as guanidinium to lyse the cells. Further processing typically involves mechanical shearing of the DNA, phenol and chloroform extraction, and ethanol or LiCl precipitation of the RNA from the guanidinium. Additional methods of mRNA preparation use oligo-dT (i.e., polythymine) immobilized on resins to capture poly-A mRNA.

It would be advantageous in diagnostic methods that detect mature cytoplasmic RNA to be able to permeabilize cells to release mature RNA species into the extraction buffer, while not appreciably releasing nuclear material. Thus, DNA and immature nuclear RNA species would not be mixed with the desired target nucleic acid. By precluding initial mixing of desired RNA species and contaminants that contain the same or complementary sequences and/or increase viscosity, additional steps needed to eliminate chromosomal DNA and resulting false positives may be avoided. A rapid, simple lysis method that will liberate cytoplasmic RNA species while not significantly releasing pre-mRNA or DNA, and that does not require a high level of specialized skill or training is particularly desirable for use in commercial assays and kits. There exists a need for a rapid, easy lysis method that yields RNA suitable for qualitative and/or quantitative nucleic acid amplification or direct detection of specific nucleic acids.

### Summary of the Invention

The present invention is directed to assay methods for detecting fusion nucleic acids, particularly chimeric mRNA species, in a biological sample.

According to one aspect of the invention, there is provided a method for detecting a fusion nucleic acid in a sample including the steps of providing a sample containing a first single-stranded fusion nucleic acid that includes a bcr-abl splice junction site; contacting under nucleic acid amplification conditions the first single-stranded fusion nucleic acid, a first promoter primer that hybridizes specifically to the fusion-nucleic acid at a first primer-binding site located 3' to the splice junction site and in an abl sequence, and at least one nucleic acid polymerase activity. Then, the method includes amplifying the fusion nucleic acid in a nucleic acid amplification reaction using the first promoter primer to produce a plurality of second nucleic acid strands complementary to at least a portion of the first single-stranded fusion nucleic acid that contains the splice junction site. Each second nucleic acid strand includes a complementary splice junction site, a first probe-binding site in a bcr sequence located 3' to and not overlapping the complementary splice junction site, and a second probe-binding site in an abl sequence located 5' to and not overlapping the complementary splice junction site, wherein the second probe-binding site overlaps or is located 3' to sequence complementary to the first primer-binding site. The method then includes the steps of hybridizing the second nucleic acid strands under hybridization conditions with an oligonucleotide probe that hybridizes specifically to the first or the second probe-binding site, thereby forming a probe:target hybrid, and detecting the probe:target hybrid in a homogeneous assay format as an indication of the presence of the fusion nucleic acid in the sample. In one embodiment of the method, the first single-stranded fusion nucleic acid is an mRNA, the polymerase activity is an RNA polymerase activity, and the oligonucleotide probe is of the same sense as the mRNA and hybridizes specifically to the first probe-binding site. In another embodiment of the method, the first single-stranded fusion nucleic acid is an mRNA, the second nucleic acid strands are complementary RNA, and the amplifying step further includes contacting the second nucleic acid strands with a second primer or promoter primer that hybridizes specifically to a second primer-binding site in a bcr sequence located 3' to both the complementary splice junction and the first probe-binding site, and uses an RNA polymerase activity, a DNA-directed DNA polymerase activity and an RNA-directed DNA polymerase activity in synthesizing amplification products. In one embodiment, the oligonucleotide probe hybridizes specifically to the second probe-binding site and is incapable of forming a stable hybridization complex with the first single-stranded fusion nucleic acid. The method may also include, in the first step, preparing cytoplasmic RNA from enkaryotic cells. in the sample by contacting a biological sample comprising the fusion nucleic acid with a solution containing a buffer, having a pH in a range of about 6,5 to about 8,5, a soluble salt having an ionic strength in the range of about 150 mM to about 1 M, a chelating agent at a concentration of about 5 mM, and a non-ionic detergent in the range of about 0,5% to about 1,5% (v/v), thereby releasing RNA from cells present in the sample. The step of preparing the sample may further include the steps of contacting the sample with a solid support which is joined to an immobilized oligonucleotide that includes a nucleotide base sequence that under hybridization conditions forms, directly or indirectly, a stable hybridization complex with a single-stranded fusion nucleic acid present in the sample, and separating the hybridization complex joined to the solid support from other sample components. In preferred embodiments, the purified fusion nucleic acid is mRNA and the nucleotide base sequence of the immobilized oligonucleotide includes a poly-T sequence. In another embodiment of the method, the fusion nucleic acid is a fusion mRNA transcript produced from a genetic t (9; 22) translocation that links two chromosomal regions from chromosome 9 and chromosome 22 at a splice junction site; in the contacting step, the first promoter primer hybridizes specifically to the fusion mRNA transcript at a first primer-binding site derived from a first chromosomal region in an abl sequence that is located 3' to the splice junction site; and in the amplifying step, the first probe-binding site is derived from a second chromosomal region in a bcr sequence, and the second probe-binding site is derived from a third chromosomal region in an abl sequence that overlaps or is located 3' to sequence complementary to the first primer-binding site; and; in the hybridizing step, the oligonucleotide probe hybridizes to the second nucleic acid strands at the first or the second probe-binding site but is incapable of hybridizing to the fusion mRNA transcript. In one embodiment for detecting fusion mRNA transcripts, the amplifying step uses a first primer that is a promoter primer and an enzyme having an RNA polymerase activity, and the hybridizing step uses an oligonucleotide probe that hybridizes specifically to the first probe-binding site. In another embodiment, the amplifying step includes a second primer or promoter primer that under amplification conditions hybridizes specifically to a nucleotide sequence of an RNA complementary to the fusion RNA transcript and uses an RNA polymerase activity, a DNA-directed DNA polymerase activity, and an RNA-directed DNA polymerase activity. In a preferred embodiment, the RNA-directed DNA polymerase activity and DNA-directed DNA polymerase activity are supplied by a reverse transcriptase. The method may also include detection of an internal control transcript, in which the amplifying step further includes amplifying an internal control transcript by using the first promoter primer that also hybridizes specifically to the internal control transcript, then the hybridizing step further includes a second oligonucleotide probe that hybridizes specifically to a sequence complementary to the internal control transcript thereby forming in internal control hybridization complex, and the detecting step further includes detecting the internal control hybridization complex. Generally, the first and second probe-binding sites may be derived from different regions of one chromosome, or the first probe-binding site is derived from a first chromosome and the second probe-binding site is derived from a second chromosome. For example, a fusion mRNA transcript may result from a translocation of human chromosomes selected from the group consisting of: t(1;19), t(2;5), t(2;13), t(4;11), t(6;9), t(8;21), t(9;11), t(9;22), t(11;14), t(11;19), t(11;22), t(12;21), t(14;18) and t(15;17). According to the present invention, the fusion mRNA transcript results from a t(9;22) translocation and the oligonucleotide probe includes a bcr-derived sequence or an abl-derived sequence. In another preferred embodiment, the oligonucleotide probe binds specifically to a bcr-derived nucleotide base sequence in the second nucleic acid strands. The invention also includes one or more oligonucleotides for use in the method of detecting a fusion mRNA transcript, having a sequence selected from the group consisting of SEQ ID NO:9 to SEQ ID NQ:16, SEQ ID NO:26 and SEQ ID NO:27. Preferably, the second primer for use in the method for detecting a fusion nucleic acid in a sample has the sequence of SEQ ID NO:5. Furthermore, it is preferred that the second oligonucleotide for use in the method for detecting a fusion nucleic acid in a sample has the sequence of SEQ ID NO:16.

The present invention further involves in a preferred embodiment in the providing step in the method for detecting a fusion nucleic acid in a sample a procedure of preparing RNA from a sample that includes the steps of providing a sample containing unpurified RNA, mixing the sample with a solution that contains a buffer having a pH in the range of about 6.5 to about 8.5, a soluble salt having an ionic strength of at least about 150 mM, a non-ionic detergent in an effective amount sufficient to release cytoplasmic RNA from a cell without causing increased viscosity in the sample due to release of chromosomal DNA from the cell, and a solid support which is joined to an immobilized oligonucleotide containing a nucleotide base sequence that hybridizes, directly or indirectly, to RNA present in the sample, thereby producing under hybridizing conditions a stable hybridization complex. Then the procedure includes the steps of separating the hybridization complex joined to the solid support from other sample components, washing the hybridization complex joined to the solid support with a washing solution having sufficient ionic strength to maintain the hybridization complex joined to the solid support, and recovering the hybridization complex joined to the solid support from the washing solution. In preferred embodiments, the sample is uncoagulated blood, plasma or bone marrow, or a suspension of eukaryotic cells. In other embodiments, the effective amount of the non-ionic detergent is between about 0.5% to about 1.5% (v/v).

The invention is more fully described in the detailed description that follows, with preferred embodiments illustrated in the figures and examples.

### Brief Description of the Figures

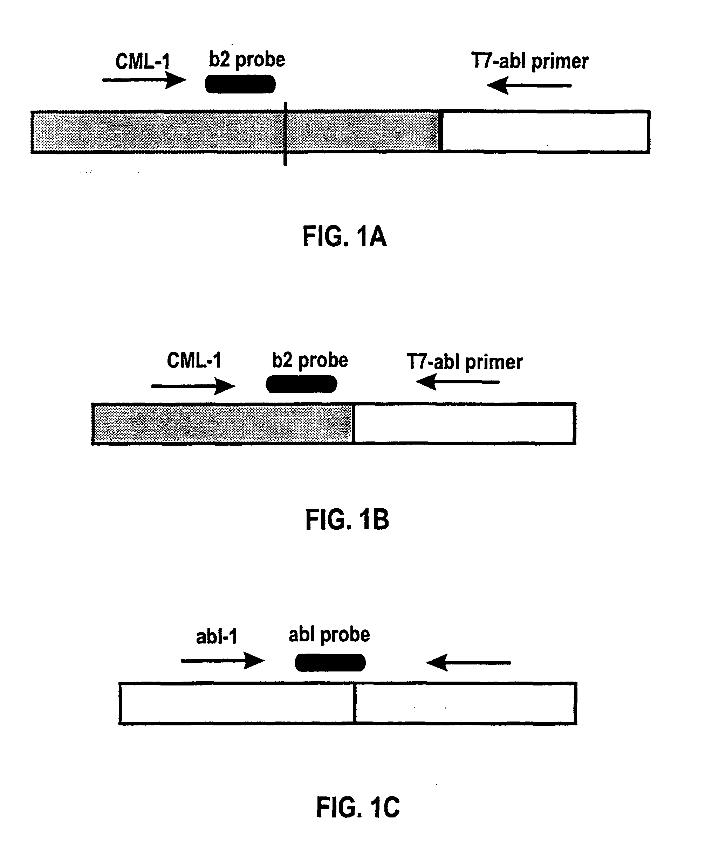

FIG. 1A is a schematic depiction of detection of a fusion transcript produced from a translocation between the *bcr* b3 region and the *abl* gene, by using a T7 *abl* promoter primer ("T7-abl primer") of the opposite sense to the target nucleic acid and a primer that is substantially identical to a sequence in the *bcr* b2 region ("CML-1") for amplification and a probe that is substantially identical to a sequence within *bcr* b2 ("b2 probe"). The dark portion of the bar to the left of the vertical line represents the *bcr* b2 sequence, the dark portion to the right of the vertical line represents the *bcr* b3 sequence, and the light portion represents *abl* sequence.
FIG. 1 B is a schematic depiction of detection of a fusion transcript produced from a translocation between the *bcr* b2 region and the *abl-*1 gene using the same combination of primers and probe as in FIG. 1A; the dark portion of the bar represents *bcr* b2 sequence and the light portion represents *abl* sequence.
FIG. 1C is a schematic depiction of detection of the normal *abl* mRNA using a T7 *abl* promoter primer ("T7-abl primer") of the opposite sense to the target nucleic acid and an "abl-1" primer of the same sense as the target nucleic acid, and an "abl probe" that is substantially identical to an *abl* target sequence, here shown at a location overlapping a potential breakpoint junction (shown as a vertical line in the bar) in the *abl* sequence.

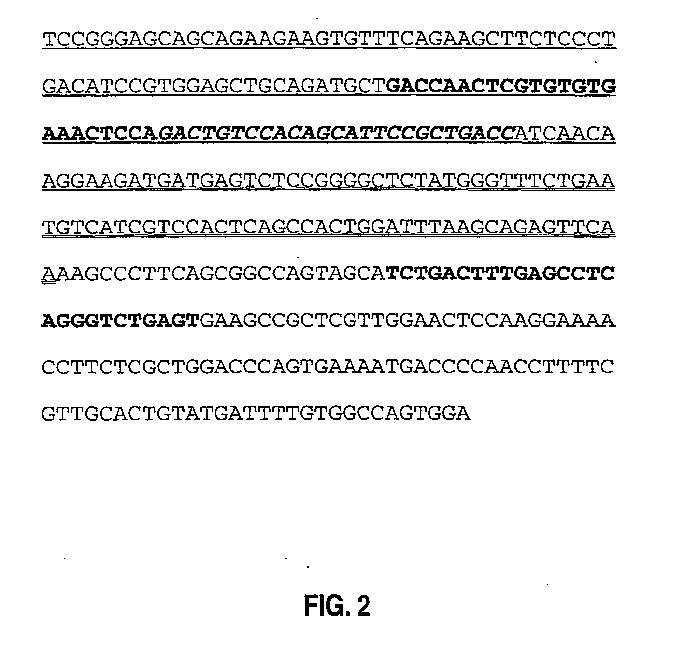

FIG. 2 shows the 5' to 3' DNA base sequence of the region surrounding the *bcr-abl* splice junction, as shown schematically in FIG. 1A, where the underlined region (residues 1 to 126) represents the *bcr b2* sequence containing the sequence complementary to a primer-binding site (bolded residues 65 to 88) and the sequence complementary to the b2 probe-binding site(bolded and italicized residues 89 to 113). The double underlined region (residues 127 to 201) represents the *bcr* b3 sequence, the splice junction occurs between bases 201 and 202, and the remaining sequence is the A2 region of *abl* containing the *abl* primer-binding site (bolded).

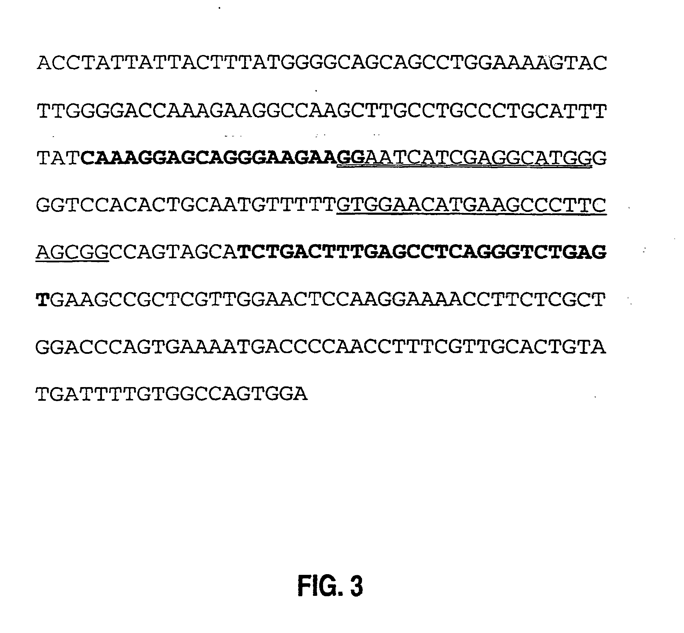

FIG. 3 shows the 5' to 3' DNA base sequence of the region surrounding a potential splice junction in a normal *abl* transcript where: residues 1 to 151 are *abl* 1b exon sequence containing a region complementary to an *abl* primer-binding site (residues 84-103, bolded); the double-underlined region (residues 102 to 119) is the complement of an *abl*-specific probe-binding site flanking the splice junction of *ablb*1 and *ablb2;* the underlined region (residues 142 to 165) is the complement of a second probe-binding site that overlaps potential splice junctions; and residues 175 to 201 (bolded) are normal *abl* sequence containing another primer-binding site.

### Detailed Description of the Invention

In a preferred embodiment, the present invention includes in the providing step in the method for detecting a fusion nucleic acid in a sample a procedure of preparing RNA samples derived from biological samples, such as body fluids, tissue or eukaryotic cells. This relatively simple procedure of RNA preparation provides RNA suitable for analysis and detection of mRNA species that occur in relatively low abundance in biological samples. The present invention also includes methods for detecting chimeric RNA species, particularly mRNA species that occur in relatively low abundance in RNA samples prepared from biological sources. These methods, useful in the human medical and veterinary fields, are useful for medical diagnoses and clinical monitoring of a patient's response to therapy where a disease or medical condition is associated with a particular type and/or level of mRNA present in a biological sample.

In addition to the definitions provided elsewhere in the specification, the following terms have the following meanings unless indicated otherwise. Other scientific and technical terms used herein have the same meaning as commonly understood by those skilled in the relevant art. General definitions of many of the terms used herein are provided, for example, in Dictionary of Microbiology and Molecular Biology, 2nd ed. (Singleton et al., 1994, John Wiley & Sons, New York, NY), The Harper Collins Dictionary of Biology (Hale & Marham, 1991, Harper Perennial, New York, NY), and Dorland's Illustrated Medical Dictionary, 27th ed. (W.A. Dorland, 1988, W.B. Saunders Co., Philadelphia, PA).
By "nucleotide sequence" is meant the sequence of nitrogenous bases along a linear information-containing molecule that is able to hydrogen-bond with a DNA or RNA having a complementary base sequence. The term is not meant to limit such information-containing molecules to polymers of nucleotides, but also includes molecules containing one or more nucleotide analogs. For example, analogs may contain subunits containing a sugar moiety or substitute other than ribose or deoxyribose (e.g.,2'halide- or methoxy-substituted pentose sugars), and/or known linkages other than phosphodiester linkages (e.g., phosphorothioate, methylphosphonate, and peptide linkages). A hydrogen-bonded nucleic acid duplex contains two complementary nucleic acid strands. These related strands are said to be of opposite "sense", in that the nucleotide sequence of either of two perfectly complementary strands automatically dictates the nucleotide sequence of the other strand, even though the nucleotide sequence of each strand differs from the other. Conventionally, one strand is arbitrarily designated as the (+) strand and the other related strand is designated as the (-) strand.

By "oligonucleotide" is meant a polymeric chain of at least two, generally between about five and about 100, chemical subunits, each subunit comprising a nucleotide base moiety, a sugar moiety, and a linking moiety that joins the subunits in a linear spacial configuration. Common nucleotide base moieties are guanine (G), adenine (A), cytosine (C), thymine (T) and uracil (U), although other rare or modified nucleotide bases able to hydrogen bond are well known to those skilled in the art. Common sugar moieties are ribose and deoxyribose, although 2'-O-methyl ribose, halogenated sugars, and other modified and different sugars are known in the art. Usually, the linking group is a phosphorus-containing moiety, most commonly a phosphodiester linkage, although other linkages, such as, for example, phosphorothioates, methylphosphonates, and non-phosphorus-containing linkages such as peptide-like linkages are known in the art, as are oligonucleotides comprising such linkages ("peptide nucleic acids" or PNA). PNA are included within this definition of an oligonucleotide. Likewise, nucleotide base moieties may be modified, e.g., by the addition of propyne groups, so long as the modified base moiety retains the ability to form a non-covalent association with G, A, C, T or U and an oligonucleotide comprising at least one modified nucleotide base moiety is not sterically prevented from hybridizing with a single-stranded nucleic acid. An oligonucleotide has a sequence of nucleotide base moieties that provides information permitting the oligonucleotide to hybridize with a complementary nucleic acid strand.

By "nucleic acid amplification conditions" is meant environmental conditions including salt concentration, temperature (including isothermal or temperature cycling), at least one nucleic acid polymerase, nucleoside triphosphates, and cofactors that permit amplification of a target nucleic acid using a given nucleic acid amplification method. Amplification product is also referred to as "amplicon."

By "primer" is meant an oligonucleotide that binds to a region of a target nucleic acid (i.e., the primer's "target-binding region" or "target-binding site") and promotes nucleic acid amplification of the target nucleic acid. Usually, a primer has a 3' end that is extended by a nucleic acid polymerase. A promoter primer is an oligonucleotide that includes a promoter sequence located 5' to the target-binding region. Under certain circumstances the 3' end of a promoter primer, or a subpopulation of promoter primers, may be modified to block or reduce primer extension.

By "target sequence" is meant the nucleotide base sequence of a nucleic acid strand (RNA or DNA), at least a portion of which is detectable using a labeled probe, that is bounded on its 3' side by a primer-binding site, or the exactly complementary RNA or DNA base sequence.

By "RNA equivalent" is meant an RNA having the same nucleotide base sequence as a DNA, except that U is substituted for T.

A "solid support" is an essentially insoluble material, under the given solvent and temperature conditions, comprising chemical groups that join with an oligonucleotide or nucleic acid. Particularly preferred is a solid support covalently coupled to an oligonucleotide that directly or indirectly binds a target nucleic acid. When the target nucleic acid is an mRNA, the coupled oligonucleotide preferably comprises a poly-T nucleotide base sequence. Preferably, the solid support is a particle (*e.g*., bead or sphere) in the micron or submicron size range. A solid support may be made of one or more of the following materials silica, polyacrylate, polyacrylamide, a metal, polystyrene, latex, nitrocellulose, polypropylene and nylon, and preferably is attracted by a magnetic field (*e.g.*, by containing a magnetite core).

By "nucleotide sequence" is meant the linear sequence of nitrogenous bases in an information-containing molecule that hydrogen bonds with a DNA or RNA having a complementary base sequence. The term is not limited to such information-containing molecules as polymers of nucleotides but includes molecules containing one or more nucleotide analogs. Nucleotide analogs may have one or more subunits containing a sugar moiety or substitute other than ribose or deoxyribose (*e.g*., 2' halide- or methoxy-substituted pentose sugars) and/or linkages other than phosphodiester linkages (*e.g*., phosphorothioate, methylphosphonate, and peptide linkages).

By "splice junction probe" is meant that the probe contains a sequence that is sufficiently complementary to hybridize to sequences on the 5' and 3' sides of a splice point. Similarly, a "breakpoint junction probe" contains a sequence that is sufficiency complementary to hybridize to sequences on the 5' and 3' sides of a chimeric nucleic acid. That is, the probe-binding site of a splice junction probe or breakpoint junction probe spans the junction that joins sequences that normally are not contiguous.

By "splice junction site" is meant the position, in the nucleotide base sequence of a nucleic acid or oligonucleotide (or its complementary strand), at which the sequence located 5' to the splice site on one strand of the nucleic acid is derived from a first nucleic acid region and the sequence located 3' to the splice site (with reference to the same strand) is derived from a second nucleic acid region, where the first and second regions normally are not contiguous. By a "fusion" or "chimeric" nucleic acid is meant a nucleic acid or oligonucleotide that contains adjacent or contigous sequences that are derived from a first and a second nucleic acid region, where the first and second regions normally are not adjacent or contiguous in cellular DNA. Generally, chimeric nucleic acid is found in nucleic acids of abnormal cells.

In a preferred embodiment, the present invention includes in the providing step in the method for detecting a fusion nucleic acid in a sample a simplified and rapid procedure for preparation of RNA from a biological sample, particularly from the cytoplasm of eukaryotic cells, which is suitable for use in an amplification and detection assay. This procedure may also be used to prepare viral RNA from a biological sample (e.g., plasma). This procedure does not require extensive extraction, shearing of chromosomal DNA to reduce viscosity, or the use of potentially harmful reagents (e.g., phenol or chloroform) to prepare RNA. Moreover, the procedure minimizes sample preparation steps, thus limiting sample loss and variability during preparation. This increased reliably and reproducibly in RNA preparation is particularly useful for assays that detect low abundance RNA species, such as fusion or chimeric RNA species. The invention also includes a method of detecting or quantifying fusion or chimeric RNA species. This method includes an initial step of contacting, under nucleic acid amplification conditions: (1) a first single-stranded fusion nucleic acid that includes a splice junction site, (2) a first promoter primer capable of hybridising to the fusion nucleic acid at a primer-binding site located 3' to the splice junction site, and (3) at least one nucleic acid polymerase activity. Then, the method proceeds to amplifying the fusion nucleic acid in a nucleic acid amplification reaction, thereby producing multiple second nucleic acid strands, each of which includes a region complementary to a region of the initial single-stranded fusion nucleic acid that includes the splice junction site, a first probe-binding site in a bcr sequence located 3' to and not overlapping the splice junction site, and a second probe-binding site in an abl sequence located 5' to and not overlapping the splice junction site.

The second probe-binding site can overlap or be located to the 3' side of the first primer-binding site. Then, the method includes hybridizing the complementary second nucleic acid strand under hybridization conditions with an oligonucleotide probe capable of hybridizing to the first or the second probe-binding site, thereby forming a probe:target hybrid (i.e., a hybridization complex formed by complementary base pairing between the probe's base sequence and the target's base sequence). The probe:target hybrid is detected in a homogeneous assay format as an indication of the presence of the fusion nucleic acid in the sample from which the single-stranded fusion nucleic acid was obtained. In this method, if the fusion nucleic acid is RNA, the obligonucleotide probe is of the same sense as the single-stranded fusion nucleic acid. If only one primer (or promoter primer) is used in the amplifying step, then the probe is targeted to the first probe-binding site.

It is important to note that this method does not use nested primers or require serial amplification reactions. Preferably, the method uses a transcription-mediated amplification system that was previously described in detail in U.S. Pat. Nos. 5,480,784, 5,399,491 and 5,554,516 to Kacian *et al.* In one embodiment of the present method, two oligonucleotides are used in the amplification procedure: one promoter primer of opposite sense than the fusion RNA to be detected and one primer of the same sense as the fusion RNA in a location 5' to both the binding site of the promoter primer and the splice junction.

As used herein, a "promoter primer" is an oligonucleotide that contains at least two distinct nucleotide base sequences. The first-sequence is the primer sequence that hybridizes to a binding site of the fusion RNA at a location 3' to the splice junction. The second sequence is a promoter sequence, located 5' to the primer sequence, that provides a preferred binding site for an RNA polymerase to begin transcription when the promoter sequence is double-stranded. Thus, the promoter sequence allows transcription of the nucleotide base sequence to which the promoter primer is hybridized *(i.e.,* RNA synthesis using the chimeric RNA as a template). An example of a promoter primer is SEQ ID NO:1 in which residues 1 to 27 provide a functional T7 promoter sequence when made double stranded, and residues 28 to 54 provide a primer sequence that binds to a complementary nucleic acid base sequence.

In another embodiment, the assay uses a single amplification oligonucleotide that is a promoter primer. When a single promoter primer is employed with no primer of the opposite sense, the chimeric RNA is detected using a probe of the same sense as the chimeric RNA that hybridizes with a nucleotide base sequence region located 3' to the splice junction on the amplified complementary nucleic acid. In this manner, only the amplified chimeric RNA is detected. Nucleic acid amplification methods employing a single promoter primer have been described in detail in U.S. Pat. No. 5,554,516 to Kacian *et al.*

Preferably, the primers (including promoter primers) and probes are directed to regions that are normally discontinuous within the chromosomal DNA. For example, in a preferred embodiment of the method, the regions comprise nucleotide base sequences that are normally present on different chromosomes. Likewise, for detecting nucleic acids that comprise regions derived from both viral (*i.e.*, provirus) and eukaryotic nucleic acids, the sequences contained in these individual regions are normally not continuous. Also, the regions to be detected may be those that are normally present on separate parts of the same chromosome, but are so widely separated that they would not be co-amplified by common amplification methods in the absence of a splicing or intrachromosomal translocation event.

In an embodiment in which two primers are used, the detection probe may be directed to a nucleotide base sequence located on either side of the splice junction site. In this aspect of the present invention, the probe must be of the same sense as the chimeric RNA. The amplified opposite sense nucleic acids are detected, thereby permitting discrimination of "normal" mRNA (which is not amplified) from abnormally spliced RNA (which has been amplified). Furthermore, because the probe is not directed to the splice junction, but instead binds to a flanking sequence located outside the breakpoint or splice region, a single probe can detect multiple spliced forms which may result from different splicing events.

By "flanking" the site of translocation is meant that the oligonucleotide binding sites are located at positions on either side of the splice junction or the breakpoint cluster region. In many diseases and conditions associated with chromosomal translocations, there may be multiple possible species of chimeric RNA. In each chimeric RNA, a known splice junction has a particular nucleotide sequence to which a probe may specifically bind, but this type of detection requires inclusion of potentially many splice junction probes, each directed to a known chimeric RNA species, both common and rarer ones.

Many characterized translocations have breakpoints clustered in discrete, known regions that are associated with the given disease or condition. To detect a translocation as a marker for a given condition or disease, it is not necessary to detect or characterize each species of chimeric RNA. Instead, using the present method, it is only necessary to detect whether an RNA associated with a translocation event has been produced by the target cell and amplified.

In a preferred embodiment, the ability of the target mRNA to be amplified indicates that a translocation has occurred in a cell. In another embodiment, such as in the amplification method employing a single promoter primer, both normal and spliced transcripts are amplified, but the probe does not hybridize to the nucleic acids amplified from the normal transcript. In either case, detection of the amplified RNA may use any of a variety of known methods such as gel electrophoresis, increased light absorption, hyperchromatic shift, or use of a detectable probe, preferably a labeled oligonucleotide probe. Suitable labels include enzymes, enzyme substrates, fluorescent, luminescent, chemiluminescent and electrochemiluminescent molecules, radionuclides, and fluorescent atoms. Preferred labels are a fluorescent or chemiluminescent label, and more preferably an acridinium ester.

The probes may be targeted to any region of the amplified nucleic acid, so long as the probe hybridizes specifically to the amplified nucleic acid. Preferred probes detect sequences outside of the splice junction (*i.e.*, flanking probes), thus increasing the number of differently spliced nucleic acids that may be detected using this method.

An example of such an assay is schematically illustrated in FIGS.1A to 1C, which show two fusion target nucleic acids (FIGS: 1A and 1B) and a normal target nucleic acid (FIG. 1C). Detection of the normal target nucleic acid is included as an optional internal control for the amplification and detection method. Referring to FIGS.1A and 1B, the fusion target nucleic acids (represented by the darkened and open bars) are amplified using primers and the amplicons are detected with a probe directed to a nucleotide base sequence flanking the splice junction (the junction of the darkened and open bars) to indicate the presence of the spliced nucleic acid in the biological sample. This assay format is capable of detecting any of a family of potential spliced nucleic acids, because the probe does not bind to the spliced junction. FIG. 1C shows an internal control (or "non-target" nucleic acid) in an assay where a normal target nucleic acid is also amplified and detected in the sample. The internal control indicates that the nucleic acids in the sample were capable of being amplified and detected by a probe *(i.e.,* the absence of contaminants that would inhibit one or both of these assay steps).

As illustrated in FIGS. 1A and 1B, amplification of target nucleic acids need not be confined to a single size or category of splicing event. In FIGS. 1A and 1B, the target nucleic acids are fusion products of two different *bcr*-*abl* translocations, one joining *bcr* b3 with *abl* (FIG. 1A), and the other joining *bcr* b2 with *abl* (FIG. 1 B). The target nucleic acids are amplified using primers directed to sites on normally non-proximal portions of the nucleic acid, such as sites normally contained on different chromosomes (*e.g.*, the bcrsite located on human chromosome 22 that is recognized by the "CML-1" primer, and the *abl* site located on human chromosome 9 that is recognized by the "T7-*abl* primer").

As shown in FIGS. 1A and 1B, amplification of target nucleic acids uses one primer specific for one of the nucleic acids involved in the chimeric splicing event (e.g., the "T7-abl primer" specific for *abl* sequence) and a second primer specific to the other nucleic acid involved in the chimeric splicing event (*e.g.*, the "CML-1" primer specific for *bcr* sequence). The amplified target sequences are detected using a probe specific for one of the two sequences (*e.g.*, the "b2 probe" specific for the *bcr* partner in the splicing events shown in FIGS. 1A and 1B). This combination of primers and probe is capable of detecting more than one chimeric splicing event because the same primers can amplify different sized products that are the result of different splicing events all of which are detectable with the same probe. For example, compare the relatively long amplified product obtained using the CML-1 and T7-abl primers with the fusion product of FIG. 1A with the relatively short amplified product obtained using the same primers with the fusion product of FIG. 1B, where both amplified products are detected with the b2 probe. Thus, the fusion transcripts resulting from any of a variety of abnormal splicing events are detectable, independent of the particular sequences of the breakpoint junctions.

The assay may optionally include amplification and detection of an internal control (non-target) nucleic acid using one primer that is also used for amplification of the target nucleic acids *(e.g.,* the *T7-abl* primer in FIGS. 1A to 1C). A second primer used for internal control amplification must be different from the second primer used in amplifying target sequences and is directed to a normally proximate region on the other side of the region that may contain one or more potential splice junctions (*e.g.*, the *abl*-1 primer as shown in FIG. 1C). Preferably, this second primer is capable of amplifying the internal control nucleic acids at a site close to the breakpoint junction region to eliminate the possibility that an abnormal splicing event is mistaken for normal nucleic acids. That is, because of the second primer's proximity to the potential splice junction region, the amplification product of the internal control nucleic acid will include a sequence for probe hybridization that will often be eliminated by abnormal splicing events. As illustrated in FIG. 1C, amplification of non-target normal *abl* nucleic acid uses primers for sequences that would normally be contiguous on non-fusion nucleic acid (*e.g.*, the "abl-1 "primer and the "T7-abl primer"). A second probe is needed for detection of the amplified internal control nucleic acids (*e.g*., the "abl probe" in FIG. 1C). This probe is unique to the internal control sequence on the same side of the potential splice junction region as the non-target-specific primer.

The relationships of these sequence regions are shown in greater detail in FIGS. 2 and 3. FIG. 2 shows the 5' to 3' DNA sequence (SEQ ID NO:24) of the region surrounding a *bcr-abl* splice junction, as shown schematically in FIG. 1A, where the underlined region (residues 1 to 126) represents the *bcr b2* sequence containing the sequence complementary to the CML-1 primer-binding site (bolded residues 65 to 88; SEQ ID NO:5) and the sequence complementary to the b2 probe-binding site (bolded and italicized residues 89 to 113; SEQ ID NO:9). The double underlined region (residues 127 to 201) represents *bcr* b3 sequence containing a splice junction between residues 201 and 202. From residue 202 to the end of the sequence shown in FIG. 2 is the *abl* A2 region that contains the primer-binding site (bolded; SEQ ID NO:22) for the T7-abl primer of FIGS. 1A to 1C.

FIG. 3 shows the 5' to 3' DNA sequence (SEQ ID NO:25) surrounding a potential splice junction region in a normal *abl* DNA. Residues 1 to 151 are *abl* 1b exon sequence and contain a region complementary to the binding site (bolded residues 84 to 103; SEQ ID NO: 13) of the abl-1 primer of FIG. 1C. The double-underlined region (residues 102 to 119; SEQ ID NO:26) are complementary to a probe-binding site that flanks the splice junction region. The underlined region (residues 142 to 165; SEQ ID NO:16) includes the potential splice junctions, and is the complement of a probe-binding site for the abl probe of FIG. 1C. Residues 152 to 299 are normal *abl* sequence containing a primer-binding site (bolded residues 175 to 201; SEQ ID NO:22) for the T7-abl primer of FIGS. 1A to 1C.

The preferred amplification method used in the present invention produces an amplified nucleic acid ("amplicon") that is an RNA, and more preferably produces predominantly amplified RNA that is complementary to the target sequence *(i.e.,* of the opposite sense as the target sequence). By "predominantly" is meant at least 50% of the product, and preferably more than 55%, is complementary RNA. Thus, if the target is mRNA, which is arbitrarily designated (+) sense strand, then the amplification products are preferably (-) sense strands. Using transcription-associated amplification methods as described previously in U.S. Pat. Nos. 5,480,784 and 5,399,491, the promoter primer is of the (-) sense and the template target nucleic acid is of the (+) sense, producing amplicons that are of the (-) sense. Although the probe may be any sequence capable of binding to amplification products, preferably the probe is of the (+) sense for hybridizing to (-) sense amplification products. If a first primer is used in common for amplification of both target and internal control nucleic acids, then the same relationship of primers, amplification products and probes is also used for control nucleic acid amplification and detection.

These methods are useful for detecting any of a variety of known genetic or physiologic events that result in spliced or otherwise rearranged nucleic acids. These methods are particularly useful for detecting the presence of fusion transcripts or chimeric transcripts that result from genetic translocations, especially those associated with pathological conditions or diseases. These methods are suitable for detecting in a biological sample a variety of known translocations that are associated with cancers, particularly forms of leukemia such as, for examples, CML and ALL.

The methods of the present invention are suitable for detecting any of a variety of known translocations that occurs in humans, requiring only the design of primers and probes for known sequences involved in the translocations (reviewed in Mitelman *et al., Cytogenet. Cell. Genet.* 55:358-386, 1990). Detectable translocations include, but not limited to, t(9;22), t(4;11), particularly t(4;11)(q21;q23), t (9;11), particularly t(9;11)(p22;q23), t(11;19), particularly t(11;19)(q23;p13), t(8;21), t(1;19), particularly in pre-B cells, t(11;14), t(2;5), particularly t(2;5)(p23;q35), t(11;22), particularly t(11;22)(q24;q12), t(15;17), t(6;9), t(14;18), t(12;21) and t(2;13) translocations. Target sequences for detection of these translocations are included in sequences known in the art, as described in: Bakhshi *et al., Cell* 41:899-906, 1985; Bakhshi *et al., Proc. Natl. Acad. Sci. USA* 84:2396-2400,1987; Barr *et al., Genomics* 11:941, 1991; Chen *et al., Blood* 78:2498-2504, 1991; Cleary *et al., Proc. Natl. Acad. Sci. USA* 82:7439-7443, 1985; Cleary *et al., Cell* 47:19-28, 1986; Crescenzi *et al*., *Proc. Natl*. *Acad. Sci. USA* 85: 4869-4873, 1988; Domer *et al., Proc. Natl. Acad Sci. USA* 90: 7884-7888, 1993; Dragon-Durey *et al., Leukemia* 12(7): 1159-1162, 1998; Groffen *et al., Cell* 36:93-99, 1984; Gu *et al., Cancer Res.* 54:2327-2330, 1994; Hermans *et al., Cell* 51:33-40, 1987; Kakizuka *et al., Cell* 66:663-674,1991; Kamps *et al., Cell* 60:547-555, 1990; Kawasaki *et al., Proc. Natl. Acad. Sci. USA* 85: 5698-5702; LeBeau *et al., Leukemia* 3(12):866-870,1989; Mellentin *et al., Science* 246: 379-382, 1989; Mitelman *et al., Cytogenet. Cell. Genet.* 55(1-4):358-386, 1990; Nakamura *et al., Proc. Natl. Acad. Sci. USA* 90:4631-4635, 1993; Nourse *et al., Cell* 60: 535-545, 1990; Sacchi *et al., Science* 231:379-382, 1986; Sarris *et al., Leuk. Lymphoma* 29(5-6): 507-514, 1998; Sawyers *et al., Proc. Natl. Acad. Sci. USA* 87:563-567,1990; Selleri *et al., Proc. Natl. Acad. Sci. USA* 88:887-891, 1991; Shtivelman *et al., Nature* 315:550-554, 1985; Sooknanan *et al., Experimental Hematol.* 21:1719-1724,1993; Tkachuk *et al., Science* 250:559-562, 1990; Tsujimoto *et al., Science* 224: 1403-1406, 1984; von Lindern *et al., Mol. Cell. Biol.* 12:1687-1697, 1992; Zhao *et al., Am. J. Hum. Genet.* 47:A119 , 1980; and Zemin-VanDerPoel *et al., Proc. Natl. Acad. Sci. USA* 88:10735-10739,1991; U.S. Pat. No. 5,057,410 to Kawasaki *et al.;* U.S. Pat. No. 5,459,251 to Tsujimoto *et al.;* U.S. Pat. No. 5,538,846 to Meeker, U.S. Pat. Nos. 5,149,628, 5,198,338, 5,202,429 and 5,242,795 to Croce *et al.;* and U.S. Pat. No. 5,547,838 to Nisson *et al.*

Unless described otherwise, the techniques described herein are standard methodologies well known to those of ordinary skill in the art. The examples of embodiments that follow are provided for illustration only.

### EXAMPLE 1

### Lysis of Biological Samples and Isolation of mRNA

The following procedure was used to isolate mRNA from biological samples. In this example, blood and bone marrow cells were used individually in the lysis and mRNA isolation procedures. The procedure works equally well on other tissues if the cells are separated into individual cells or small clumps of cells using standard mincing, screen separation and/or proteolysis methods to limit the number of cells present in clumps. The lysis procedure comprises contacting a suspension of target cells with a lysing solution containing at least 150 mM of a soluble salt, preferably a lithium halide salt (*e.g*., LiCl), a chelating agent (*e.g*., ethylenediamine tetraacetic acid (EDTA)) and an amount of a non-ionic detergent effective to lyse the cytoplasmic membrane of the cell without substantially releasing the contents of the nucleus. Generally, the non-ionic detergent in the lysing solution was an octylphenoxy polyethoxyethanol (a TRITON®-type detergent), although other non-ionic detergents (*e.g*., TWEEN®-type and NP-type detergents) function equivalently. By "effective amount" of non-ionic detergent is meant an amount sufficient to cause lysis or permeabilization of the cytoplasmic membrane without causing substantial release of nuclear DNA or RNA, generally between about 0.5% (v/v) to about 2.0% (v/v). A preferred lysing solution contained about 1% (v/v) TRITON® X-102.

In a typical procedure, about 250 µl of uncoagulated blood or bone marrow was added to about 750 µl of the lysing solution. By "uncoagulated" is meant that the blood or bone marrow was treated upon collection with about 2mM to about 20 mM EDTA, or an effective amount of heparin or similar anticoagulant known in the art. The proportions of sample to lysing solution are not critical, but generally a ratio of about 1:1 to about 1:3 of the components (sample:lysing solution) is capable of lysing the sample. Generally, the lysing solution consisted of 50 mM HEPES (pH 7.5), 1 M LiCl, 5 mM EDTA, and 1 % TRITON® X-102. The pH of the buffer may be in a range above and below neutral (pH 7.0), preferably is in a pH range of about 6.5 to about 8.5, and more preferably is about pH 7.5, to facilitate capture of the mRNA by hybridization, such as described below.

Surprisingly, after mixing the sample and the lysing solution, the released RNA in the lysis mixture was stable and may be stored at room temperature for at least about 2 hours without significant degradation of the RNA, even in the absence of additional RNAse inhibitors. None of the HEPES, LiCl, EDTA or TRITON® X-102 components of the lysing solution were individually effective in preventing almost-immediate degradation of the RNA. Thus, it was surprising that the combination of components was effective in inhibiting RNA degradation.

For mRNA isolation (or target capture), capture particles were added to the above-described lysis mixture. About 30 µl of a suspension of superparamagnetic particles (approximately 300 µg) to which poly-T (in a range between dT₁₄ to dT₃₀) was linked covalently at a density of between about 1 to 100 pmoles per mg was added to about 1 ml of the lysis mixture. Particles having dT₁₄, dT₂₀, dT₂₅ or dT₃₀ oligomers have all been used in these procedures. Generally, the particles had attached between about 10 to 100 pmoles of poly-dT per mg, and most commonly the particles had attached about 10 to 50 pmoles of poly-dT per mg of particles. The particles were suspended in standard phosphate buffered saline (PBS), pH 7.4, containing 140 mM NaCl for addition to the lysis mixture.

Typically, the particles used were a magnetite core coated with latex or silica, which are commercially available, to which poly-dT oligonucleotides ("tails") were attached. Although it is not crucial that the particles be magnetic or paramagnetic, the magnetic property aids in the recovery of the particles after hybridization of the liberated mRNA onto the particles via the poly-dT tails. Those skilled in the art will appreciate that non-magnetic particles (*e.g*., cellulose) with poly-dT may be substituted and separated using standard sedimentation or filtration methods For coupling to the poly-dT, underivatized particles have free groups such as amine, hydroxyl, carboxyl, ester groups or mixtures of these groups. Appropriate particles may be obtained already derivatized with poly-dT tails (*e.g.*, Serodyn, Dynal, Novagen). Alternatively, underivatized particles may be purchased (*e.g.*, from Seradyne) and joined to poly-dT oligonucleotides using well-known coupling chemistry (Lund *et al., Nuc. Acids Res.* 16:10861-10880,1988).

The lysis mixture and the poly-dT particles were gently mixed by vortexing and then incubated at between about 22°C to 42°C for about 30 min. The particles were separated from the remainder of the mixture by application of a magnetic field to retain the particles; the supernatant was discarded. Those skilled in the art could readily substitute sedimentation (*e.g.*, centrifugation) for the magnetic separation step. Separated particles were washed in about 1 ml of a wash solution of 50 mM HEPES (pH 7.5), 5 mM EDTA, 150 mM NaCl and 0.1% (w/v) sodium dodecyl sulfate (SDS) by mixing with a vortexer for about 3 to 5 seconds to suspend the particles which were then separated from the supernatant as described above, and the supernatant wash was discarded. The washing procedure was repeated twice and the particles were finally suspended in 250 µl of a buffer consisting of 10 mM HEPES (pH 7.5) and 1 mM EDTA. This suspension was either stored at -30°C for later use or used immediately. RNA prepared in this manner has been stored frozen for over a year with no noticeable diminution in the structural or functional Integrity of the RNA.

If the isolated RNA is to be used immediately, it may be either released from the capture particles (*e.g.*, using a standard low salt elution process) or it may be amplified without releasing it from the particles by using an amplification procedure that obviates the need to elute. This is achieved, for example, by using primers that bind to regions of the isolated nucleic acid not involved in base pairing with the poly-dT or in other interactions with the solid phase matrix that might bind mRNA without releasing it. from the particles.

One skilled in the art will recognize that the exact volumes and proportions exemplified above are not critical to the invention. It is important, however, that samples derived from biological tissue susceptible to coagulation (*e.g.*, blood, bone marrow, plasma or cell suspensions) be treated to prevent coagulation. It is also important for cellular samples that the ionic strength of the lysing solution be at least about 150 mM, preferably in a range between about 150 mM to about 1M, to permit lysis of the cell membrane of a target cell without concomitant release of nuclear DNA. At ionic strengths less than 150 mM, released nuclear material contaminated the released cytoplasmic nucleic acids, for example, with chromosomal DNA. Although not wishing to be bound to a theory or mechanism, it is likely that at about 150 mM or greater ionic strength, the nuclear membrane remains sufficiently intact to prevent significant release of chromosomal DNA. Contaminating chromosomal DNA increased the viscosity of the mixture which may interfere with using the cytoplasmic RNA in a subsequent assay, for example, by cross-reacting and producing false positive reactions, by sequestering the RNA and/or primers, and/or preventing mixing of reactants. A lysing solution containing lithium salts is preferred for preventing RNA degradation, although buffers containing other soluble salts (*e.g*., NaCl) and a known RNAse inhibitor are expected to be equally effective in this selective lysis procedure so long as the ionic strength is at least 150 mM.

Nucleic acids prepared by the above-described lysis procedure can be used with other methods of selecting and immobilizing a target nucleic acid. One such method includes a mediator oligonucleotide that hybridizes in solution to a specific target nucleic acid as described in U.S. Pat. No. 4,751,177 to Stabinsky. Other immobilization methods have been described in U.S. Pat. Nos. 4,486,539 and 4,563,419 to Ranki *et al*., EP Pat. Publication No: 159719 by Rabbani *et al.* , EP Pat. Publication No. 128332 by Pergolizzi *et al.,* U.S. Pat. No. 5,476,769 to Soderlund *et al.,* U.S. Pat. No. 5,474,895 to Ishii *et al.* and EP Pat. Publication No. 444120 by Homes *et al.*

The present lysis method is useful for the preparation of nucleic acids from cells contained in a variety of tissues. For example, cells originating in solid tissue, such as solid tumor tissue, may be minced and treated with trypsin using standard methods to make a cell suspension that is then lysed as described above. Other suitable methods of putting cells into suspension are well known in the art. As shown below, cells grown in tissue culture or liquid medium may also be used.

Although mixing the reagents by vortexing is preferred for small-scale usage of the method, for large scale use, other less labor-intensive mixing methods known in the art may be advantageous. Known methods that provide thorough and vigorous mixing are within the scope of the invention.

Although three washing steps were usually used before amplifying the captured target nucleic acid, the number of washing steps following linking of the target nucleic acids to the particles may be varied. Washing steps are important, not only for removing contaminating proteins and nucleic acids from the cytoplasmic nucleic acid preparation, but also for removing lithium that has been found to inhibit at least one enzymatic activity (RNA directed DNA polymerase, DNA directed DNA polymerase, RNAse H, and RNA polymerase) used in the preferred transcription-associated amplification method (see U.S. Pat. Nos. 5, 480,784 and 5,399,491 to Kacian *et al.).* Lithium salts may not inhibit other enzymes, and thus exchanging the cation after lysis may not be necessary if other amplification methods are used.

Particles used in selection of the target nucleic acid may be separated from the supernatant by a variety of known procedures such as filtration, precipitation and centrifugation. The particles, as described above, may have nucleic acid capture probes joined thereto to bind specifically to a particular cytoplasmic nucleic acid. Alternatively, although less preferred, the solid phase may non-specifically bind the target nucleic acid, as, for example, by using polycationic supports (see U.S. Pat. No. 5,599,667 to Arnold *et al.).*

The next example describes amplification of nucleic acid that has been isolated using the lysis method of this example.

### EXAMPLE 2

### Amplification of Isolated Nucleic Acid from a Biological Sample and Detection of Amplified Product

For transcription-associated amplification of nucleic acid isolated from a biological sample, 50 µl of a particle suspension containing nucleic acids isolated, as described in Example 1, from the blood of a CML positive patent was added to a tube containing 25 µl of an amplification reagent. For each reaction, the amplification reagent contained 160 mM Tris-HCl (pH 7.5),100 mM MgCl₂ 70 mM KCI, 20% (w/v) polyvinylpyrrolidone, 16 mM each of the four ribonudeoside triphosphates ATP, GTP, CTP, and UTP, 4 mM each of the four deoxyribonucleoside triphosphates dATP, dGTP, dCTP, and dTTP, 400 nM (15 pmoles) of a promoter primer having the nucleotide base sequence of SEQ ID NO:1 (TAAATTAATACGACTCACTATAGGGAGACTCAGACCCTGAGGCTCAAAGTCAGA), and 400 nM (15 pmoles) of a primer having the nucleotide base sequence of SEQ ID NO:5 (GACCAACTCGTGTGTGAAACTCCA). After mixing, the tube was incubated for 10 min at 60°C. Generally, any temperature that is suitable to melt intramolecular base pairing in the target nucleic acid is permissible at this step. Preferably, the incubation temperature is between about 60°C and 70°C, more preferably between about 65°C and about 67°C.

Next, the tube was incubated at about 42°C for 5 min. Then, an enzyme reagent (25 µl containing 2000 units of recombinant MMLV reverse transcriptase, 2000 units recombinant T7 RNA polymerase, 8 mM HEPES (pH 7.5), 50 mM N-acetyl-L-cysteine, 0.04 mM zinc acetate, 80 mM trehalose, 140 mM Tris-HCl (pH 8.0), 70 mM KCl, 1mM EDTA, 0.01 % (w/v) phenol red, 10% (v/v) TRITON® X-102 and 20% (v/v) glycerol) was added to the reaction mixture. The tube was gently mixed and incubated for about 1 hr at 42°C. This amplification method produced amplified RNA of a sense opposite to that of the target RNA.

Amplified RNA was detected using 100 µl of a probe reagent containing 100 mM lithium succinate (pH 4.7), 1.2 M LiCl, 15 mM aldrithiol-2, 2% (w/v) lithium lauryl sulfate (LLS), 20 mM EDTA, 20 mM ethylene glycol-bis-(β-amino ethyl ether) N, N, N', N'-tetracetic acid (EGTA), 3% ethanol, and 7.5 nM of a hybridization probe labeled with a chemiluminescent acridinium ester (AE). The synthetic DNA probe specific for bcr b2-sequence detection had the sequence of SEQ ID NO: 9 (GACTGTCCACAGCATTCCGCTGACC) that was linked to the AE label using non-nucleotide linkers and methods previously described in U.S. Pat. No. 5,585,481 to Arnold *et al.* This detection solution was added to the reaction mix and incubated at 60°C for 30 min to permit hybridization of the probe to the amplified target.

The probe was detected in a homogeneous assay format, such as the homogeneous protection assay (HPA) described in detail in U.S. Pat. No. 5,283,174, to Arnold *et al.* Briefly, 300 µl of an alkaline solution containing 600 mM sodium borate (pH 8.5) and 1% (v/v) TRITON® X-100 was added to the mixture described above to hydrolyze the AE label present in unbound probe. The AE label on hybridized probe is protected from hydrolysis by its association with a double helix, whereas AE label on unhybridized probe is not protected from hydrolysis. Thus, unhybridized probe is preferentially made undetectable. The solution was incubated at 60°C for 10 min, cooled to room temperature for 5 min and mixed with 200 µl of a solution containing 30 mM hydrogen peroxide and 1 mM nitric acid, followed immediately by addition of 200 µl of a solution containing 1 M NaOH and 2% (w/v) ZWITTERGENT® 3-14. Chemiluminescence was detected using a luminometer (e.g., LEADER® 1, LEADER® 50, LEADER® 450 or LEADER® HC, all from Gen-Probe, Inc., San Diego, CA). Chemiluminescent output was measured and reported in relative light units ("RLU").

Although the method exemplified herein employs specific detection formats for detecting a nucleic acid probe, those skilled in the art could readily use labels other than AE, such as radioactive labels, fluorescent and other chemiluminescent labels to detect hybridized probe using standard methods. Likewise, although homogeneous assay systems have certain clear advantages over heterogeneous assays *(i.e.,* those necessitating physical separation to differentiate signal of hybridized probe from signal due to unhybridized probe), the homogeneous detection aspect is not critical to the method.

### EXAMPLE 3

### Amplification of Isolated Spliced Nucleic Acid and Detection of Amplified Product

In this assay, a spliced target nucleic acid resulting from a bcr-abl translocation was amplified and detected with a probe directed to a sequence flanking the splice junction to indicate the presence of the fusion product in the biological sample. The general method used for amplification and detection of the fusion product is illustrated in FIGS. 1A to 1C.

The biological sample used was blood cells obtained from patients known to be positive for CML, from which poly-A RNA was isolated using the lysis method substantially as described in Example 1. Amplification of the RNA was conducted substantially as described in Example 2, but using the promoter primer of SEQ ID NO:1, the primer of SEQ ID NO:5 and 400 nM of an *abl*-specific DNA primer having the sequence of SEQ ID NO: 13 (CAAAGGAGCAGGGAAGAAGG). This *abl*-specific primer is of the same sense as the target nucleic acid.

Prior to detection, the amplified nucleic acids were divided into two aliquots. The *bcr*-specific AE-labeled probe of SEQ ID NO:9 was added to the first aliquot for detection substantially as described in Example 2. The *bcr*-specific AE-labeled probe is functionally the same as the b2 probe described with reference to FIGS. 1A and 1B, and the fusion nucleic acid was detected by measuring RLU as described in Example 2. An AE-labeled *abl*-specific probe having the sequence of SEQ ID NO:16 (GTGGAACATGAAGCCCTTCAGCGG) was added to the second aliquot and the mixture was processed for detection of chemiluminescence substantially as described in Example 2. This *abl-*specific probe is capable of hybridizing to the human *abl* gene spanning a commonly used splice junction region, although a probe directed to 5' to the splice junction region may also be used (see Example 6). Because the primers used in this example were designed to amplify each of the fusion target and the normal *abl* nucleic acids present in the amplification mixture, they co-amplified both types of nucleic acids in the amplification reaction mixture but were separately detectable in the two aliquots of the detection step.

The chemiluminescence of each of the two aliquots was detected substantially as described in Example 2, although the volumes of the reagents was adjusted appropriately to account for the fact that the amplification mixture had been divided (i.e, half volumes were used).

Alternative procedures may be used to detect each of the target and non-target amplicons. One such alternative procedure makes use of two chemiluminescent labels having different characteristics, such as different wavelengths of light emission, different optimal reaction pH values, or different chemiluminescent reaction kinetics, which characteristics make it possible to detect two different amplicons in the same reaction vessel by employing probes differently labeled with the appropriate label. Such procedures have been previously disclosed in detail in PCT Int'l Pat. Publication No. WO 96/13612 and PCT Int'l Pat. Publication No. WO 91/00511.

### EXAMPLE 4

### Amplification Using a Single Promoter primer and Selective Detection of Amplified Nucleic Acids

This example demonstrates nucleic acid amplification using a single promoter primer capable of hybridizing to the target nucleic acid for the production of amplified nucleic acids of a sense opposite to that of the target. Both normal and fusion nucleic acids were amplified using one primer, but the amplified nucleic acids were detected separately using a *bcr* probe targeted to a position located to the 5' side (relative to the target nucleic acid) of the breakpoint junction, and an *abl* probe targeted to an *abl* sequence that would be missing in a fusion nucleic acid.

For comparison, three different sources of poly-dT magnetic particles were used in this example essentially as described in Example 1. Commercially available particles with linked 25-mer poly- dT were purchased (dT₂₅ beads, from Novagen) and two sets of synthesized particles were prepared by coupling poly-dT to underivatized particles (purchased from Seradyne). One set of beads was coupled to a homopolymeric 14-mer oligonucleotide ("dT₁₄ beads"), and a second set of beads was coupled to a homopolymeric 30-mer oligonucleotide ("dT₃₀ beads"). Each type of bead was present in a suspension substantially as described in Example 1.

Cytoplasmic mRNA was prepared from K562 cells, grown in standard tissue culture to a density of about 5 x 10⁶ cells/ml and then treated according to the sample preparation method described in Example 1. Approximately 2 x 10⁵ cells were used for each reaction mixture tested which was then used undiluted or at a 1:10 dilution. In separate reaction mixtures, 60 µl of each washed bead preparation was used per nucleic acid amplification reaction. Control samples had no beads added, to provide a background measurement (data not shown) that was subtracted from the sample results to which beads had been added. Nucleic acid amplification was performed substantially as described in Example 2 and in U.S. Pat. No. 5,554,516, to Kacian *et al.,* except that a single promoter primer of SEQ ID NO:1 (30 pmoles) was used in the amplification reaction. No primers of the same sense as the target nucleic acid were used.

Amplification reactions were allowed to proceed for one hour. Then each sample was divided into two unequal aliquots, one aliquot containing one-third of each amplification reaction for detection with the *bcr*-specific AE-labeled probe of SEQ ID NO:9, and the other aliquot containing two-thirds of the amplification reaction for detection with the ablspecific AE-labeled probe of SEQ ID NO:16. Probe hybridization was performed substantially as described in Example 2. Detection of the hybridized probes was performed substantially as described in Example 2, with the volumes of the detection reagents adjusted proportionately to the volume of sample used. The light emitted by the labels was measured in a LEADER® 50 luminometer in relative light units (RLU) as described previously and the results are shown in Table 1.

**Table 1**

| | ***bcr* probe** | | ***abl* probe** | |
|---|---|---|---|---|
| Capture Particles | undiluted | 1:10 dilution | undiluted | 1:10 dilution |
| dT₂₅ (Novagen) | 224935 | 95570 | 55807 | 21264 |
| dT₁₄ (synthesized) | 323646 | 21999 | 109303 | 7725 |
| dT₃₀ (synthesized) | 12510 | 969 | 13685 | 2607 |

These results indicate that the method of the present invention can be used in an amplification format that uses a single promoter primer that hybridizes to the target nucleic acid at a position located 3' to a potential splice junction region. Amplification resulted in accumulation of an amplicon (complementary RNA transcript) having the opposite sense as that of the target nucleic acid. The results also show that particles coupled with 14-mer poly-dT oligonucleotides and commercially-obtained (Novagen) particles having attached poly-dT₂₅ were effective under these assay conditions for capturing mRNA in solution. Synthesized poly-dT₃₀ particles were somewhat variable for RNA capture based on the results shown in Table 1. As shown by these results, an amplification reaction using a single promoter primer may be used to specifically detect the presence of two different nucleic acid transcripts in the same biological sample by using different probes. In this case, one transcript was detected by the *bcr* probe, and another was detected by the *abl* probe. Results of other experiments showed no cross reaction between these two probes. Thus, detection of one transcript (e.g., the *abl* transcript) may serve as an internal control in the sample preparation, amplification and detection steps of the method.

### EXAMPLE 5

### Amplification and Detection of Normal abl Transcripts in Peripheral Blood Cells

Amplification was carried out on nucleic acids isolated from peripheral blood cells and stored before use, substantially as described in Examples 1 and 2. Whole blood treated with EDTA was processed as described in Example 1, and the particles to which cytoplasmic RNA bound were stored before use. This example shows use of the sample preparation method to prepare normal *abl* mRNA and of the amplification method to permit the detection of normal *abl* mRNA. This example also shows that different sources of immobilized dT, having different dT lengths, are effective for target isolation.

The particles used for the isolation of the naturally occurring *abl* gene product were the same types as used in Example 4, and the isolation of the cytoplasmic nucleic acids was performed substantially as described in Example 1. The blood specimens were used undiluted or at a dilution of 1:10 or 1:100. The particles with captured target nucleic acid (60 µg and 6 µg) were used in the transcription-mediated amplification procedure substantially as described in Example 2, using a promoter primer having the sequence of SEQ ID NO:1 and a primer having the sequence of SEQ ID NO:13. Following amplification, the amplification products were detected using an *abl*-specific probe having the sequence of SEQ ID NO:16, using the detection procedures substantially as described in Example 2. Chemiluminescence results, measured in RLU, are shown in Table 2. The negative control ("No Target") represents background RLU.

**Table 2**

| DETECTION OF DIFFERENT TARGET AMOUNTS WITH *abl* PROBE | | | | |
|---|---|---|---|---|
| Capture Particles | undiluted | 1:10 dilution | 1:100 dilution | No Target |
| 60 µg of dT₂₅ (Novagen) | 247,314 | 258,311 | 37,434 | 2,181 |
| 6 µg of dT₂₅ (Novagen) | 19,737 | 10,273 | 2,987 | 1,779 |
| 60 µg of dT₁₄ (synthesized) | 957,6685 | 264,429 | 21,984 | 1,744 |
| 6 µg of dT₁₄ (synthesized) | 67,992 | 14,455 | 2,532 | 1,780 |
| 60 µg of dT₃₀ (synthesized) | 440,204 | 79,873 | 5,135 | 1,548 |
| 6 µg of dT₃₀ (synthesized) | 61,618 | 15,568 | 2,369 | 1,807 |

The data of Table 2 show that the present sample preparation method is suitable to capture specific nucleic acid species which can then be amplified with target-specific primers and detected using a target-specific probe.

### EXAMPLE 6

### Amplification and Detection of Normal abl Transcripts and Fusion bcr-abl Transcripts in RNA isolated from CML Patients

This example demonstrates that *bcr-abl* transcripts of two different types *(bcr b2-abl* and *bcr b3-abl)* can be detected using different bcr probes in amplification products of RNA obtained from CML patients. It further shows that amplification and detection of *abl* RNA serves as an internal control.

The sources of RNA used in these experiments were: (1) CML patients' RNA obtained from three individuals, (2) control synthetic RNA transcripts produced by standard *in vitro* transcription methods from an *abl* gene clone (cloned from a patient's RNA using standard cDNA cloning methods), and (3) synthetic RNA transcripts produced a clone containing a *bcr b3-abl* translocation breakpoint junction (cloned from the K562 cell line). The three CML patients (Patients A, B and C) exhibited different phases of the disease: Patients A and B had active CML symptoms, and Patient C was assayed after receiving a bone marrow transplant and displayed little or no CML symptoms. For each patient, total RNA was isolated from the buffy coat of blood samples using a standard guanidinium isothiocyanate method (Chirgwin, *et al., 1979, Biochem.* 18:5294-5299). The total RNA used for each assay for each patient was about 10 ng (equivalent to about 1,000 cells). The control synthetic RNA were assayed using numbers of copies of the sequence calculated using standard procedures, with the copy number per assay shown in Table 3. The negative controls had no RNA added to the amplification reaction mixtures.

Amplification of the patients' total RNA and the control synthetic RNA transcripts was performed essentially as described in Example 2. Detection of the amplified nucleic acids was done essentially as described in Example 2, individually detecting an amplification aliquot using a *bcrb2* probe (an AE-labeled oligonucleotide having SEQ ID NO:9), a *bcr b3* probe (an AE-labeled oligonucleotide having SEQ ID NO:27), and an *abl* probe (an AE-labeled oligonucleotide having SEQ ID NO:26). The *abl* probe is targeted to a region shown in FIG. 3 (double underlined sequence). The assays were performed in triplicate and the average (mean) RLU results are presented in Table 3 ("ND" means "not done").

**Table 3**

| RNA Specimen | *abl* Probe (SEQ ID NO:26) | *bcr* b2 Probe (SEQ ID NO:9) | *bcr* b3 Probe (SEO ID ND:27) |
|---|---|---|---|
| Patient A | 928,274 | 6,166,770 | 3,200,051 |
| Patient B | 95,173 | 4,843,570 | 1,547 |
| Patient C | 3,250,592 | 3,304 | ND |
| 20,000 copies *abl* | 4,676,542 | ND | ND |
| 2,000 copies *abl* | 1,298,834 | ND | ND |
| 200 copies *abl* | 130,249 | ND | ND |
| 2,000 copies *bcr b3-ab*l | ND | 6,012,846 | 3,195,019 |
| 50 copies *bcr b3-ab*l | ND | 2,756,934 | 729,648 |
| No RNA | 2,305 | 3,344 | 1,591 |

The results of Table 3 show that the *bcr* b2-specific probe was able to detect as few as 50 copies of *bcr*b2 (see column 3, data line 8) and detected the presence of *bcr*b2 in RNA from Patients A and B, but not from Patient C (column 3, data lines 1 to 3, compared to the "No RNA" control of column 3, data line 9). The results also show that the *bcr* b3 probe was able to detect as few as 50 copies of *bcr* b3 (see column 4, data line 8) and detected the presence of *bcr* b3 in RNA from Patient A, but not in RNA from Patient B. Patient C would not be expected to have *bcr* b3 because the translocation in that patient had already removed the target of the *bcr* b2 probe, and therefore would also have removed the target of the *bcr* b3 probe. Table 3 also shows that the internal control, *abl,* was detected in all three parents, presumably representing detection of normal *abl* transcripts (column 2, data lines 1-3). Other results (not shown) have confirmed that the *bcr* b2 and *bcr* b3 probes do not cross-react with *abl* amplicons and the *abl* probe does not cross-react with *bcr* amplicons.

## Claims

1. A method for detecting a fusion nucleic acid in a sample, comprising the steps of:
a) providing a sample containing a first single-stranded fusion nucleic acid comprising a *bcr-abl* splice junction;
b) contacting under nucleic acid amplification conditions:
the first single-stranded fusion nucleic acid,
a first promoter primer that hybridizes specifically to the first single-stranded fusion nucleic acid at a first primer-binding site located 3' to the splice junction site and in an *abl* sequence, and
at least one nucleic acid polymerase activity;
c) amplifying the first single-stranded fusion nucleic acid in a nucleic acid amplification reaction using the first primer to produce a plurality of second nucleic acid strands complementary to at least a portion of the first single-stranded fusion nucleic acid, wherein each second nucleic acid strand comprises:
a complementary splice junction site,
a first probe-binding site in a *bcr* sequence located 3' to and not overlapping the complementary splice junction site, and
a second probe-binding site in an *abl* sequence located 5' to and not overlapping the complementary splice junction site, wherein the second probe-binding site overlaps or is located 3' to sequence complementary to the first primer-binding site;
d) hybridizing the second nucleic acid strands under hybridization conditions with an oligonucleotide probe that hybridizes specifically to either the first probe-binding site or the second probe-binding site but not both sites, thereby forming a probe:target hybrid; and
e) detecting the probe:target hybrid in a homogeneous assay format as an indication of the presence of the first single-stranded fusion nucleic acid in the sample.

2. The method of Claim 1, wherein the first single-stranded fusion nucleic acid is an mRNA, the polymerase activity comprises an RNA polymerase activity, and the oligonucleotide probe is of the same sense as the mRNA and hybridizes specifically to the first probe-binding site.

3. The method of Claim 1 or 2,
wherein the first single-stranded fusion nucleic acid is a mRNA,
wherein the second nucleic acid strands are complementary RNA, and
wherein the amplifying step further includes contacting the second nucleic acid strands with a second primer or promoter primer that hybridizes specifically to a second primer-binding site in a bcr sequence located 3' to both the complementary splice junction site and the first probe-binding site, and uses an RNA polymerase activity, a DNA-directed DNA polymerase activity and an RNA-directed DNA polymerase activity in synthesizing amplification products.

4. The method of any one of claims 1 to 3, wherein the oligonucleotide probe hybridizes specifically to the second probe-binding site and is incapable of forming a stable hybridization complex with the first single-stranded fusion nucleic acid.

5. The method of any one of claims 1 to 4, wherein the providing step includes preparing cytoplasmic RNA from eukaryotic cells in the sample by
contacting a biological sample containing the first single-stranded fusion nucleic acid in cells with a solution comprising:
a buffer having a pH in a range of about 6.5 to about 8.5,
a soluble salt having an ionic strength in a range of about 150 mM to about 1 M,
a chelating agent at a concentration of about 5 mM, and
a non-ionic detergent in a range of about 0.5% to about 1.5% (v/v),
thereby releasing cytoplasmic RNA from the cells present in the sample without extraction or use of phenol or chloroform reagents, and
then mixing with a solid support which is joined to an immobilized oligonucleotide comprising a nucleotide base sequence that hybridizes, directly or indirectly, to cytoplasmic RNA released from the cells, thereby producing under hybridizing conditions a stable hybridization complex;
separating the hybridization complex joined to the solid support from other sample components;
washing the hybridization complex joined to the solid support with a washing solution having sufficient ionic strength to maintain the hybridization complex joined to the solid support; and
recovering the hybridization complex joined to the solid support from the washing solution.

6. The method of any one of claims 1 to 5, wherein the providing step includes
contacting the sample with a solid support which is joined to an immobilized oligonucleotide comprising a nucleotide base sequence that under hybridization conditions forms, directly or indirectly, a stable hybridization complex with the first single-stranded fusion nucleic acid present in the sample; and
separating the stable hybridization complex joined to the solid support from other sample components, thereby separating the first single-stranded fusion nucleic acid without extraction or use of phenol or chloroform reagents.

7. The method of any one of claims 1 to 6, wherein
in the providing step, the first single-stranded fusion nucleic acid is a fusion mRNA transcript produced from a genetic t(9;22) translocation that links two chromosomal regions from chromosome 9 and chromosome 22 at a splice junction site;
in the contacting step, the first promoter primer hybridizes specifically to the fusion mRNA transcript at the first primer-binding site which is derived from a first chromosomal region in an *abl* sequence and is located 3' to the splice junction site;
in the amplifying step, the first probe-binding site is derived from a second chromosomal region in a bcr sequence, and the second probe-binding site is derived from a third chromosomal region in an *abl* sequence that overlaps or is located 3' to a sequence complementary to the first primer-binding site;
in the hybridizing step, the oligonucleotide probe hybridizes specifically to the second nucleic acid strands at either the first probe-binding site or the second probe-binding site, but is incapable of hybridizing to the fusion mRNA transcript, thereby forming a hybridization complex of the probe and the second nucleic acid strand; and
in the detecting step, the hybridization complex is detected in a homogeneous assay format as an indication of the presence of the fusion mRNA transcript in the sample.

8. The method of any one of claims 1 to 7, wherein
the amplifying step further includes amplifying an internal control transcript by using the first promoter primer that also hybridizes specifically to the internal control transcript,
the hybridizing step uses a second oligonucleotide probe that hybridizes specifically to a sequence complementary to the internal control transcript, thereby forming an internal control hybridization complex, and
the detecting step further includes detecting the presence of the internal control hybridization complex.

9. The method of any one of claims 1 to 8, wherein the amplifying step includes a second primer or promoter primer that under amplification conditions hybridizes specifically to a nucleotide sequence of an RNA complementary to the first single-stranded fusion nucleic acid.

10. The method of any one of claims 1 to 2, wherein the first single-stranded fusion nucleic acid is a fusion mRNA transcript derived from a t(9;22) translocation and the oligonucleotide probe binds specifically to a *bcr* -derived sequence or an *abl*-derived sequence but not both sequences.

11. A second primer for use in the method of any one of claims 3 to 10 having a sequence of SEQ ID NO:5.

12. An oligonucleotide probe for use in the method of any one of claims 1 to 10, having the sequence of SEQ ID NO:9, SEQ ID NO:16, SEQ ID NO:26, or SEQ ID NO:27.

13. The second oligonucleotide probe for use in the method of claim 8 having the sequence of SEQ ID NO:16.

## Patentansprüche

1. Ein Verfahren zum Detektieren einer Fusionsnukleinsäure in einer Probe, welches die Schritte umfasst:
a) Bereitstellen einer Probe, die eine erste einzelsträngige Fusionsnukleinsäure enthält, die eine *bcr*-*abl*-Spleißverbindung umfasst;
b) In-Kontakt-Bringen unter Nukleinsäureamplifizierungsbedingungen:
der ersten einzelsträngigen Fusionsnukleinsäure, eines ersten Promotor-Primers, der spezifisch mit der ersten einzelsträngigen Fusionsnukleinsäure an einer ersten Primer-Bindungsstelle hybridisiert, die 3' zur Spleißverbindungsstelle und in einer *abl*-Sequenz angeordnet ist, und
zumindest einer Nukleinsäure-Polymeraseaktivität;
c) Amplifizieren der ersten einzelsträngigen Fusionsnukleinsäure in einer Reaktion zur Nukleinsäureamplifizierung unter Verwendung des ersten Primers zur Herstellung einer Vielzahl von zweiten Nukleinsäuresträngen, die zumindest mit einem Abschnitt der ersten einzelsträngigen Fusionsnukleinsäure komplementär sind, wobei jeder zweite Nukleinsäurestrang umfasst:
eine komplementäre Spleißverbindungsstelle,
eine erste Sonden-Bindungsstelle in einer *bcr*-Sequenz, die 3' angeordnet ist und die nicht mit der komplementären Spleißverbindungsstelle überlappt, und
eine zweite Sonden-Bindungsstelle in einer
abl-Sequenz, die 5' angeordnet ist und die nicht mit der komplementären Spleißverbindungsstelle überlappt, wobei die zweite Sonden-Bindungsstelle überlappt oder 3' zur Sequenz angeordnet ist, die komplementär zur erste Primer-Bindungsstelle ist;
d) Hybridisieren des zweiten Nukleinsäurestranges unter Hybridisierungsbedingungen mit einer Oligonukleotidsonde, die spezifisch entweder mit der ersten Sonden-Bindungsstelle oder mit der zweiten Sonden-Bindungsstelle hybridisiert, jedoch nicht mit beiden Stellen, wodurch ein Sonden:Target-Hybrid ausgebildet wird; und
e) Detektieren des Sonden:Target-Hybrids in einem homogenen Assayformat als ein Nachweis auf die Anwesenheit der ersten einzelsträngigen Fusionsnukleinsäure in der Probe.

2. Das Verfahren gemäß Anspruch 1, wobei die erste einzelsträngige Fusionsnukleinsäure eine mRNA ist, die Polymeraseaktivität eine RNA-Polymeraseaktivität umfasst und die Oligonukleotidsonde den gleichen Sinn aufweist wie die mRNA und spezifisch mit der ersten Sonden-Bindungsstelle hybridisiert.

3. Das Verfahren gemäß Anspruch 1 oder 2,
wobei die erste einzelsträngige Fusionsnukleinsäure eine mRNA ist,
wobei die zweiten Nukleinsäurestränge aus komplementärer RNA bestehen und,
wobei der Amplifizierungsschritt weiter das In-Kontakt-Bringen der zweiten Nukleinsäurstränge mit einem zweiten Primer oder Promotor-Primer, der spezifisch mit einer zweiten Primer-Bindungstelle in einer *bcr*-Sequenz, die 3' sowohl zur komplementären Spleißverbindungsstelle als auch zur ersten Sonden-Bindungsstelle angeordnet ist, hybridisiert und eine RNA-Polymeraseaktivität, eine DNA-gerichtete
DNA-Polymeraseaktivität und eine RNA-gerichtete
DNA-Polymeraseaktivität beim Synthetisieren von
Amplifizierungsprodukten verwendet, beinhaltet.

4. Das Verfahren gemäß einem der Ansprüche 1 bis 3, wobei die Oligonukleotidsonde spezifisch mit der zweiten Sonden-Bindungsstelle hybridisiert und nicht in der Lage ist, einen stabilen Hybridisierungskomplex mit der ersten einzelsträngigen Fusionsnukleinsäure auszubilden.

5. Das Verfahren gemäß einem der Ansprüche 1 bis 4, wobei der Bereitstellungsschritt das Zubereiten cytoplasmatischer RNA aus eukaryotischen Zellen in der Probe durch
In-Kontakt-Bringen einer biologischen Probe, welche die erste einzelsträngige Fusionsnukleinsäure in Zellen mit einer Lösung beinhaltet, die umfasst:
einen Puffer mit einem pH in einem Bereich von etwa 6.5 bis 8.5,
ein lösliches Salz mit einer Ionenstärke in einem Bereich von etwa 150 mM bis etwa 1 M,
ein chelatbildendes Agens mit einer Konzentration von etwa 5 mM, und
ein nicht-ionisches Detergens in einem Bereich von etwa 0.5% bis etwa 1.5% (v/v),
wodurch cytoplasmatische RNA aus den in der Probe vorhandenen Zellen ohne Extraktion oder Verwendung von Phenol oder Chloroformreagenzien freigesetzt wird, und
anschließendes Vermengen mit einem Trägermaterial, das mit einem immobilisierten Oligonukleotid verbunden ist, das eine Nukleotidbasensequenz umfasst, die direkt oder indirekt mit cytoplasmatischer RNA hybridisiert, die von den Zellen freigesetzt wird, wodurch unter Hybridisierungsbedingungen ein stabiler Hybridisationskomplex hergestellt wird;
Abtrennen des mit dem Trägermaterial verbundenen Hybridisationskomplexes von anderen Probenbestandteilen;
Waschen des mit dem Trägermaterial verbundenen Hybridisationskomplexes mit einer Waschlösung, die eine ausreichende Ionenstärke aufweist, damit der an dem Trägermaterial gebundene Hybridisationskomplex erhalten bleibt; und
Wiedergewinnen des am Trägermaterial gebundenen Hybridisationskomplexes aus der Waschlösung, beinhaltet.

6. Das Verfahren gemäß einem der Ansprüche 1 bis 5, wobei der Bereitstellungsschritt
das In-Kontakt-Bringen der Probe mit einem Trägermaterial, das mit einem immobilisierten Oligonukleotid verbunden ist, das eine Nukleotidbasensequenz umfasst, die unter Hybridisierungsbedingungen direkt oder indirekt einen stabilen Hybridisationskomplex mit der in der Probe vorhandenen ersten einzelsträngigen Fusionsnukleinsäure ausbildet; und
das Abtrennen des mit dem Trägermaterial verbundenen stabilen Hybridisationskomplexes von anderen Probenbestandteilen, wobei die erste einzelsträngige Fusionsnukleinsäure ohne Extraktion oder Verwendung von Phenol oder Chloroformreagenzien abgetrennt wird, beinhaltet.

7. Das Verfahren gemäß einem der Ansprüche 1 bis 6, wobei
im Bereitstellungsschritt die erste einzelsträngige Fusionsnukleinsäure ein Fusions-mRNA-Transkript ist, das aus einer genetischen t(9;22) Translokation hergestellt worden ist, die zwei chromosomale Bereiche des Chromosoms 9 und Chromosoms 22 an einer Spleißverbindungsstelle miteinander verbindet;
im Schritt des In-Kontakt-Bringens der erste Promotor-Primer spezifisch mit dem Fusions-mRNA-Transkript an der ersten Primer-Bindungsstelle, die von einem ersten chromosomalen Bereich in einer *abl-*Sequenz abgeleitet und 3' zu einer Spleißverbindungsstelle angeordnet ist, hybridisiert;
im Amplifizierungsschritt die erste Sonden-Bindungsstelle von einem zweiten chromosomalen Bereich in einer *bcr*-Sequenz abgeleitet ist, und die zweite Sonden-Bindungsstelle von einem dritten chromosomalen Bereich in einer *abl*-Sequenz abgeleitet ist, die überlappt mit oder 3' zu einer Sequenz angeordnet ist, die zur ersten Primer-Bindungsstelle komplementär ist;
im Hybridisierungsschritt die Oligonukleotidsonde spezifisch mit den zweiten Nukleinsäuresträngen entweder an der ersten Sonden-Bindungsstelle oder an der zweiten Sondenbindungsstelle hybridisiert, jedoch nicht in der Lage ist, mit dem Fusions-mRNA-Transkript zu hybridisieren, wodurch ein Hybridisationskomplex der Sonde und des zweiten Nukleinsäurestranges ausgebildet wird; und
im Detektionsschritt der Hybridisationskomplex in einem homogenen Assayformat als ein Nachweis auf die Anwesenheit des Fusions-mRNA-Transkripts in der Probe detektiert wird.

8. Das Verfahren gemäß einem der Ansprüche 1 bis 7, wobei
der Amplifizierungsschritt weiter das Amplifizieren eines internen Kontroll-Transkripts unter Verwendung des ersten Promotor-Primers, der ebenfalls spezifisch mit dem internen Kontroll-Transkript hybridisiert, beinhaltet,
der Hybridisationsschritt eine zweite Oligonukleotidsonde, die spezifisch mit einer Sequenz hybridisiert, die komplementär zum internen Kontroll-Transkript ist, wodurch ein interner Kontroll-Hybridisationskomplex ausgebildet wird, verwendet, und
der Detektionsschritt weiter das Detektieren der Anwesenheit des internen Kontroll-Hybridisationskomplexes mit einschließt.

9. Das Verfahren gemäß einem der Ansprüche 1 bis 8, wobei der Amplifizierungsschritt einen zweiten Primer oder Promotor-Primer, der unter Bedingungen zur Amplifizierung spezifisch mit einer Nukleotidsequenz einer RNA hybridisiert, die komplementär zur ersten einzelsträngigen Fusionsnukleinsäure ist, beinhaltet.

10. Das Verfahren gemäß einem der Ansprüche 1 bis 2, wobei die erste einzelsträngige Fusionsnukleinsäure ein von einer t(9;22) Translokation abgeleitetes Fusions-mRNA-Transkript ist und die Oligonukleotidsonde spezifisch mit einer von bcrabgeleiteten Sequenz oder mit einer von *abl*-abgeleiteten Sequenz, jedoch nicht mit beiden Sequenzen, bindet.

11. Ein zweiter Primer zur Verwendung im Verfahren gemäß einem der Ansprüche 3 bis 10 mit einer Sequenz der SEQ ID Nr.:5.

12. Eine Oligonukleotidsonde zur Verwendung im Verfahren gemäß einem der Ansprüche 1 bis 10 mit der Sequenz der SEQ ID NR.:9, SEQ ID Nr.:16, SEQ ID Nr.:26 oder SEQ ID Nr.:27.

13. Die zweite Oligonukleotidsonde zur Verwendung im Verfahren gemäß Anspruch 8 mit der Sequenz der SEQ ID Nr.:16.

## Revendications

1. Procédé de détection d'un acide nucléique de fusion dans un échantillon, comprenant les étapes consistant à :
a) fournir un échantillon contenant un premier acide nucléique de fusion simple brin comprenant une jonction d'épissage *bcr-abl ;*
b) mettre en contact dans des conditions d'amplification des acides nucléiques :
le premier acide nucléique de fusion simple brin,
une première amorce de promoteur qui s'hybride spécifiquement avec le premier acide nucléique de fusion simple brin au niveau d'un premier site de liaison à l'amorce localisé en 3' du site de jonction d'épissage et dans une séquence *abl,* et
au moins une activité acide nucléique polymérase ;
c) amplifier le premier acide nucléique de fusion simple brin dans une réaction d'amplification des acides nucléiques en utilisant la première amorce pour produire une pluralité de seconds brins d'acides nucléiques complémentaires d'au moins une partie du premier acide nucléique de fusion simple brin, où chaque second brin d'acide nucléique comprend
un site de jonction d'épissage, complémentaire,
un premier site de liaison à la sonde dans une séquence *bcr* localisée en 3' du site de jonction d'épissage complémentaire, et ne chevauchant pas celui-ci, et
un second site de liaison à la sonde dans une séquence *abl* localisée en 5' du site de jonction d'épissage complémentaire et ne chevauchant pas celui-ci, où le second site de liaison à la sonde chevauche, ou est localisé en 3' de la séquence complémentaire du premier site de liaison à l'amorce ;
d) hybrider les seconds brins d'acides nucléiques dans des conditions d'hybridation avec une sonde oligonucléotidique qui s'hybride spécifiquement soit au premier site de liaison à la sonde soit au second site de liaison à la sonde mais pas aux deux sites, et forme ainsi un hybride sonde/cible ; et
e) détecter l'hybride sonde/cible une présentation de test homogène en tant qu'indication de la présence du premier acide nucléique de fusion simple brin dans l'échantillon.

2. Procédé selon la revendication 1, où le premier acide nucléique de fusion simple brin est un ARNm, l'activité polymérase comprend une activité ARN polymérase, et la sonde oligonucléotidique est dans le même sens que l'ARNm et s'hybride spécifiquement au premier site de liaison à la sonde.

3. Procédé selon la revendication 1 ou 2, où
le premier acide nucléique de fusion simple brin est un ARNm,
les seconds brins d'acides nucléiques sont de l'ARN complémentaire,
l'étape d'amplification inclut en outre la mise en contact des seconds brins d'acides nucléiques avec une seconde amorce ou une amorce de promoteur qui s'hybride spécifiquement à un second site de liaison à l'amorce dans une séquence *bcr,* localisée en 3' du site de jonction d'épissage complémentaire et du premier site de liaison à la sonde, et l'utilisation d'une activité ARN polymérase, une activité ADN polymérase spécifique de l'ADN et une activité ADN polymérase spécifique de l'ARN pour synthétiser les produits d'amplification.

4. Procédé selon l'une quelconque des revendications 1 à 3, où la sonde oligonucléotidique s'hybride spécifiquement au second site de liaison à la sonde et est incapable de former un complexe d'hybridation stable avec le premier acide nucléique de fusion simple brin.

5. Procédé selon l'une quelconque des revendications 1 à 4, où l'étape préliminaire inclut la préparation d'un ARN cytoplasmique à partir de cellules eucaryotes dans l'échantillon en
mettant en contact un échantillon biologique contenant le premier acide nucléique de fusion simple brin dans des cellules avec une solution comprenant :
un tampon d'un pH compris dans une gamme d'environ 6,5 à environ 8,5 ;
un sel soluble d'une force ionique comprise dans une gamme d'environ 150 mM à environ 1 M,
un agent chélateur à une concentration d'environ 5 mM et
un détergent non ionique dans une gamme d'environ 0,5% à environ 1,5% (volume/volume),
de manière à libérer l'ARN cytoplasmique des cellules présentes dans l'échantillon sans avoir recours à une extraction ni à l'utilisation de réactifs de type phénol ou chloroforme, et
en le mélangeant ensuite avec un support solide qui est lié à un oligonucléotide immobilisé comprenant une séquence de bases nucléotidiques qui s'hybride, directement ou indirectement, avec l'ARN cytoplasmique libéré par les cellules, produisant ainsi, dans des conditions d'hybridation, un complexe d'hybridation stable ;
en séparant le complexe d'hybridation lié au support solide des autres composants de l'échantillon ;
en lavant le complexe d'hybridation lié au support solide avec une solution de lavage d'une force ionique suffisante pour maintenir la liaison du complexe d'hybridation au support solide ; et
en récupérant le complexe d'hybridation lié au support solide à partir de la solution de lavage.

6. Procédé selon l'une quelconque des revendications 1 à 5, où l'étape préliminaire inclut :
la mise en contact de l'échantillon avec un support solide qui est lié à un oligonucléotide immobilisé comprenant une séquence de bases nucléotidiques qui forme, directement ou indirectement, dans des conditions favorables à l'hybridation, un complexe d'hybridation stable, avec le premier acide nucléique de fusion simple brin présent dans l'échantillon ; et
la séparation du complexe d'hybridation stable lié au support solide des autres composants de l'échantillon, de manière à séparer le premier acide nucléique de fusion simple brin sans avoir recours à une extraction ni à l'utilisation de réactifs de type phénol ou chloroforme.

7. Procédé selon l'une quelconque des revendications 1 à 6, où dans l'étape préliminaire, le premier acide nucléique de fusion simple brin est un transcrit d'ARNm de fusion produit à partir d'une translocation génétique t(9;22) qui lie deux régions chromosomiques du chromosome 9 et du chromosome 22 au niveau du site de jonction d'épissage ;
dans l'étape de mise en contact, la première amorce de promoteur s'hybride spécifiquement au transcrit d'ARNm de fusion au niveau du premier site de liaison à l'amorce qui est dérivé d'une première région chromosomique dans une séquence *abl* et est localisé en 3' du site de jonction d'épissage ;
dans l'étape d'amplification, le premier site de liaison à la sonde est dérivé d'une seconde région chromosomique dans une séquence *bcr* et le second site de liaison à la sonde est dérivé d'une troisième région chromosomique dans une séquence abl qui chevauche ou est localisée en 3' d'une séquence complémentaire du premier site de liaison à l'amorce ;
dans l'étape d'hybridation, la sonde oligonucléotidique s'hybride spécifiquement aux seconds brins d'acides nucléiques au niveau du premier site de liaison à la sonde ou du second site de liaison à la sonde, mais est incapable de s'hybrider au transcrit d'ARNm de fusion, de manière à former un complexe d'hybridation de la sonde et du second brin d'acide nucléique ; et
dans l'étape de détection, le complexe d'hybridation est détecté dans une présentation de test homogène en tant que signe indicateur de la présence du transcrit d'ARNm dans l'échantillon.

8. Procédé selon l'une quelconque des revendications 1 à 7, où l'étape d'amplification inclut de surcroît l'amplification d'un transcrit témoin interne en utilisant la première amorce de promoteur qui s'hybride également spécifiquement avec le transcrit témoin interne,
l'étape d'hybridation utilise une seconde sonde oligonucléotidique qui s'hybride spécifiquement à une séquence complémentaire du transcrit témoin interne, de manière à former un complexe d'hybridation témoin interne, et
l'étape de détection comprend également la détection de la présence du complexe d'hybridation témoin interne.

9. Procédé selon l'une quelconque des revendications 1 à 8, où l'étape d'amplification inclut une seconde amorce ou une amorce de promoteur qui, dans des conditions d'amplification, s'hybride spécifiquement avec une séquence nucléotidique d'un ARN complémentaire du premier acide nucléique de fusion simple brin.

10. Procédé selon l'une quelconque des revendications 1 à 2, où le premier acide nucléique de fusion simple brin est un transcrit d'ARNm de fusion dérivé d'une translocation génétique t(9;22) et la sonde oligonucléotidique se lie spécifiquement à une séquence dérivée de *bcr* ou une séquence dérivée de *abl* mais pas aux deux séquences.

11. Seconde amorce à utiliser dans le procédé selon l'une quelconque des revendications 3 à 10 possédant une séquence de la SEQ ID n°5.

12. Sonde oligonucléotidique destinée à être utilisée dans le procédé selon l'une quelconque des revendications 1 à 10 possédant la séquence de la SEQ ID n°9, de la SEQ ID n°16, de la SEQ ID n°26, de la SEQ ID n°27.

13. Seconde sonde oligonucléotidique destinée à être utilisée dans le procédé de la revendication 8 possédant la séquence de la SEQ ID n°16.


### SEQUENCE LISTING

<110> GEN-PROBE INCORPORATED
<120> METHODS FOR DETECTING AND MEASURING SPLICED NUCLEIC ACIDS
<130> GP091PCT
<140> PCT/US99/16832
<141> 1999-07-23
<150> US 09/121,239
<151> 1998-07-23
<160> 27
<170> PatentIn Ver. 2.1
<210> 1
<211> 54
<212> DNA
<213> Artificial Sequence
<220>
<223> Description of Artificial Sequence: Synthetic promoter primer including T7 promoter sequence at residues 1-27
<400> 1
   taaattaata cgactcacta tagggagact cagaccctga ggctcaaagt caga 54
<210> 2
<211> 54
<212> RNA
<213> Artificial Sequence
<220>
<223> Description of Artificial Sequence: RNA version of SEQ ID NO: 1
<400> 2
   uaaauuaaua cgacucacua uagggagacu cagacccuga ggcucaaagu caga 54
<210> 3
<211> 54
<212> DNA
<213> Artificial Sequence
<220>
<223> Description of Artificial Sequence:Reverse complement of SEQ ID NO:1
<400> 3
   tctgactttg agcctcaggg tctgagtctc cctatagtga gtcgtattaa ttta 54
<210> 4
<211> 54
<212> RNA
<213> Artificial Sequence
<220>
<223> Description of Artificial Sequence: RNA version of SEQ ID NO:3
<400> 4
   ucugacuuug agccucaggg ucugagucuc ccuauaguga gucguauuaa uuua 54
<210> 5
<211> 24
<212> DNA
<213> Artificial Sequence
<220>
<223> Description of Artificial Sequence: Synthetic Primer
<400> 5
   gaccaactcg tgtgtgaaac tcca 24
<210> 6
<211> 24
<212> RNA
<213> Artificial Sequence
<220>
<223> Description of Artificial Sequence: RNA version of SEQ ID NO:5
<400> 6
   gaccaacucg ugugugaaac ucca 24
<210> 7
<211> 24
<212> DNA
<213> Artificial Sequence
<220>
<223> Description of Artificial Sequence: Reverse complement of SEQ ID NO:5
<400> 7
   tggagtttca cacacgagtt ggtc 24
<210> 8
<211> 24
<212> RNA
<213> Artificial Sequence
<220>
<223> Description of Artificial Sequence: RNA version of SEQ ID NO:7
<400> 8
   uggaguuuca cacacgaguu gguc 24
<210> 9
<211> 25
<212> DNA
<213> Artificial Sequence
<220>
<223> Description of Artificial Sequence: Probe for bcr b2 sequence
<400> 9
   gactgtccac agcattccgc tgacc 25
<210> 10
<211> 25
<212> RNA
<213> Artificial Sequence
<220>
<223> Description of Artificial Sequence: RNA version of
   SEQ ID NO:9
<400> 10
   gacuguccac agcauuccgc ugacc 25
<210> 11
<211> 25
<212> DNA
<213> Artificial Sequence
<220>
<223> Description of Artificial Sequence: Reverse complement of SEQ ID NO:9
<400> 11
   ggtcagcgga atgctgtgga cagtc 25
<210> 12
<211> 25
<212> RNA
<213> Artificial Sequence
<220>
<223> Description of Artificial Sequence: RNA version of SEQ ID NO:11
<400> 12
   ggucagcgga augcugugga caguc 25
<210> 13
<211> 20
<212> DNA
<213> Artificial Sequence
<220>
<223> Description of Artificial Sequence: Primer sequence for abl-1
<400> 13
   caaaggagca gggaagaagg 20
<210> 14
<211> 20
<212> DNA
<213> Artificial Sequence
<220>
<223> Description of Artificial Sequence: Reverse complement of SEQ ID NO:13
<400> 14
   ccttcttccc tgctcctttg 20
<210> 15
<211> 20
<212> RNA
<213> Artificial Sequence
<220>
<223> Description of Artificial Sequence: RNA version of SEQ ID NO:14
<400> 15
   ccuucuuccc ugcuccuuug 20
<210> 16
<211> 24
<212> DNA
<213> Artificial Sequence
<220>
<223> Description of Artificial Sequence: Probe for abl sequence
<400> 16
   gtggaacatg aagcccttca gcgg 24
<210> 17
<211> 24
<212> RNA
<213> Artificial Sequence
<220>
<223> Description of Artificial Sequence: RNA version of SEQ ID NO:16
<400> 17
   guggaacaug aagcccuuca gcgg 24
<210> 18
<211> 24
<212> DNA
<213> Artificial Sequence
<220>
<223> Description of Artificial Sequence: Reverse complement of SEQ ID NO:16
<400> 18
   ccgctgaagg gcttcatgtt ccac 24
<210> 19
<211> 24
<212> RNA
<213> Artificial Sequence
<220>
<223> Description of Artificial Sequence: RNA version of SEQ ID NO:18
<400> 19
   ccgcugaagg gcuucauguu ccac 24
<210> 20
<211> 27
<212> DNA
<213> Artificial Sequence
<220>
<223> Description of Artificial Sequence: Primer sequence as in SEQ ID NO:1 but without T7 promoter sequence
<400> 20
   actcagaccc tgaggctcaa agtcaga 27
<210> 21
<211> 27
<212> RNA
<213> Artificial Sequence
<220>
<223> Description of Artificial Sequence: RNA version of
   SEQ ID NO:20
<400> 21
   acucagaccc ugaggcucaa agucaga 27
<210> 22
<211> 27
<212> DNA
<213> Artificial Sequence
<220>
<223> Description of Artificial Sequence: Reverse complement of SEQ ID NO:20
<400> 22
   tctgactttg agcctcaggg tctgagt 27
<210> 23
<211> 27
<212> RNA
<213> Artificial Sequence
<220>
<223> Description of Artificial Sequence: RNA version of SEQ ID NO:22
<400> 23
   ucugacuuug agccucaggg ucugagu 27
<210> 24
<211> 350
<212> DNA
<213> Homo sapiens
<400> 24
   tccgggagca gcagaagaag tgtttcagaa gcttctccct gacatccgtg gagctgcaga 60
   tgctgaccaa ctcgtgtgtg aaactccaga ctgtccacag cattccgctg accatcaaca 120
   aggaagatga tgagtctccg gggctctatg ggtttctgaa tgtcatcgtc cactcagcca 180
   ctggatttaa gcagagttca aaagcccttc agcggccagt agcatctgac tttgagcctc 240
   agggtctgag tgaagccgct cgttggaact ccaaggaaaa ccttctcgct ggacccagtg 300
   aaaatgaccc caaccttttc gttgcactgt atgattttgt ggccagtgga 350
<210> 25
<211> 299
<212> DNA
<213> Homo sapiens
<400> 25
   acctattatt actttatggg gcagcagcct ggaaaagtac ttggggacca aagaaggcca 60
   agcttgcctg ccctgcattt tatcaaagga gcagggaaga aggaatcatc gaggcatggg 120
   ggtccacact gcaatgtttt tgtggaacat gaagcccttc agcggccagt agcatctgac 180
   tttgagcctc agggtctgag tgaagccgct cgttggaact ccaaggaaaa ccttctcgct 240
   ggacccagtg aaaatgaccc caacctttcg ttgcactgta tgattttgtg gccagtgga  299
<210> 26
<211> 18
<212> DNA
<213> Artificial Sequence
<220>
<223> Description of Artificial Sequence: Probe for abl
<400> 26
   ggaatcatcg aggcatgg                                               18
<210> 27
<211> 26
<212> DNA
<213> Artificial Sequence
<220>
<223> Description of Artificial Sequence: Probe for bcr b3
<400> 27
   cactcagcca ctggatttaa gcagag                                      26